# EUROPEAN PATENT APPLICATION

(11) **EP 4 206 222 A1**
(43) Date of publication of application: **05.07.2023**
(21) Application number: 21860507.9
(22) Date of filing: 27.08.2021
(51) Int. Cl.: C07K 16/00, C07K 14/00, C12N 15/00, A61K 39/00, C12P 21/08

(54) **SIGNAL PEPTIDE FOR REDUCING END HETEROGENEITY OF HETEROLOGOUS POLYPEPTIDE**

(30) Priority: 28.08.2020 CN 202010882873; 20.08.2021 CN 202110958128
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: ZHOU, Songtao, Shanghai 200245 (CN); LIU, Xun, Shanghai 200245 (CN)
(74) Representative: Sonzogni, Laura Gabriella
(86) International application number: PCT/CN2021/114911
(87) International publication number: WO 2022/042673

(57) **Abstract**

The present invention relates to a signal peptide for reducing the end heterogeneity of a heterologous polypeptide. Specifically, the present invention relates to a signal peptide for protein expression. The present invention further relates to a recombinant polypeptide, a preparation method therefor, and a composition containing a recombinant polypeptide.

## Description

### TECHNICAL FIELD

The present disclosure relates to the field of molecular biology and protein engineering, and in particular to a method for reducing terminal heterogeneity of a heterologous polypeptide.

### BACKGROUND

The statements herein merely provide background information related to the present disclosure and may not necessarily constitute the prior art.

Signal peptides are short peptide chains that direct the transfer of newly synthesized proteins to the secretory pathway. The signal peptide is located at the N-terminus of the secreted protein. It generally consists of 15-30 amino acids. It usually comprises three regions: a positively charged N-terminus, which is referred to as the basic amino terminal; an intermediate hydrophobic sequence, which is mainly composed of neutral amino acids and capable of forming an α helix structure and is the main functional region of the signal peptide; and a longer, negatively charged C-terminus, which contains small amino acids, is the signal sequence cleavage site and is also known as the processing region. When synthesized, the signal peptide sequence is recognized by a signal recognition particle (SRP), and the protein synthesis is suspended or slowed. The signal recognition particle carries the ribosome onto the endoplasmic reticulum, and the protein synthesis is resumed. Under the guidance of the signal peptide, the newly synthesized protein enters the lumen of the endoplasmic reticulum, and the signal peptide sequence is cleaved under the action of a signal peptidase.

Generally, efficient cleavage of the signal peptide ensures that both the light and heavy chain polypeptides of the antibody can be accurately cleaved, which is very important for mammalian cell-mediated antibody expression. However, during cleavage of the signal peptide, the cleavage site may be changed, resulting in extension or truncation of the light and heavy amino acid chains of the recombinant antibody. For a truncated or extended antibody, its structural changes will result in changes in affinity, thereby affecting antibody activity; therefore, the extension or truncation of the amino acid chain needs to be avoided during antibody production. In addition, a part of the products was truncated or extended, so that the product quality varies from batch to batch in the production process, additional quality control is required, and such a problem is difficult to solve technically. Therefore, it is currently a common consensus in the industry to produce light and heavy chain antibodies with N-terminal homogeneity.

### SUMMARY

The present disclosure relates to a signal peptide and use thereof, wherein the signal peptide is used to reduce terminal heterogeneity of a heterologous polypeptide.

The present disclosure provides a method for reducing the N-terminal heterogeneity of a heavy chain of an antibody and/or a light chain of an antibody, which comprises culturing a host cell, wherein the host cell comprises: (1) a first polynucleotide encoding the heavy chain of the antibody and a first signal peptide operably linked to the N-terminus of the heavy chain; wherein the first signal peptide comprises an amino acid sequence of SEQ ID NO: 55 or SEQ ID NO: 56; and/or
(2) a second polynucleotide encoding the light chain of the antibody and a second signal peptide operably linked to the N-terminus of the light chain; wherein the second signal peptide comprises an amino acid sequence of SEQ ID NO: 55 or SEQ ID NO: 56; and
expressing the heavy chain of the antibody and/or the light chain of the antibody;
wherein, the SEQ ID NO: 55 is set forth in MEWSWVFLFFLSLTGX₁HX₂, wherein X₁ is V, A, S, T, G, C, L or I, and X₂ is A, G, S, C, T or Q;
the SEQ ID NO: 56 is set forth in MSVPTQVLGLLLLWLTDX₃RX₄, wherein X₃ is V, A, S, T, G, C, L or I, X₄ is A, G, S, C, T or Q, and X₄ is not C when X₃ is selected from A.

In some embodiments, provided is the method for reducing N-terminal heterogeneity of a heavy chain of an antibody and/or a light chain of an antibody described above, wherein the heavy chain of the antibody obtained through expression has a terminal extension ratio of less than 3%, 2.5%, 2%, 1.5% or 1%, and/or the light chain of the antibody obtained through expression has a terminal extension ratio of less than 3%, 2.5%, 2%, 1.5% or 1%; in some embodiments, the heavy chain of the antibody obtained through expression has a terminal extension ratio of less than 1%, and/or the light chain of the antibody obtained through expression has a terminal extension ratio of less than 1%. In some embodiments, the terminal extension ratio is measured based on a peptide mapping assay. In some embodiments, the heavy chain of the antibody obtained through expression is substantially free of residual terminal residues, and/or the light chain of the antibody obtained through expression is substantially free of residual terminal residues; in some embodiments, the heavy chain of the antibody obtained through expression has a terminal extension ratio of 0%, and/or the light chain of the antibody obtained through expression has a terminal extension ratio of 0%.

In some embodiments, provided is the method for reducing N-terminal heterogeneity of a heavy chain of an antibody and/or a light chain of an antibody described above, wherein the first signal peptide or the second signal peptide each independently comprises a peptide selected from the group consisting of:
SEQ ID NO: 57: MEWSWVFLFFLSLTGVHA;
SEQ ID NO: 58: MEWSWVFLFFLSLTGVHG;
SEQ ID NO: 59: MEWSWVFLFFLSLTGVHS;
SEQ ID NO: 60: MEWSWVFLFFLSLTGVHC;
SEQ ID NO: 61: MEWSWVFLFFLSLTGVHT;
SEQ ID NO: 62: MEWSWVFLFFLSLTGVHQ;
SEQ ID NO: 63: MEWSWVFLFFLSLTGAHA;
SEQ ID NO: 64: MEWSWVFLFFLSLTGAHG;
SEQ ID NO: 65: MEWSWVFLFFLSLTGAHS;
SEQ ID NO: 66: MEWSWVFLFFLSLTGAHC;
SEQ ID NO: 67: MEWSWVFLFFLSLTGAHT;
SEQ ID NO: 68: MEWSWVFLFFLSLTGAHQ;
SEQ ID NO: 69: MEWSWVFLFFLSLTGSHA;
SEQ ID NO: 70: MEWSWVFLFFLSLTGSHG;
SEQ ID NO: 71: MEWSWVFLFFLSLTGSHS;
SEQ ID NO: 72: MEWSWVFLFFLSLTGSHC;
SEQ ID NO: 73: MEWSWVFLFFLSLTGSHT;
SEQ ID NO: 74: MEWSWVFLFFLSLTGSHQ;
SEQ ID NO: 75: MEWSWVFLFFLSLTGTHA;
SEQ ID NO: 76: MEWSWVFLFFLSLTGTHG;
SEQ ID NO: 77: MEWSWVFLFFLSLTGTHS;
SEQ ID NO: 78: MEWSWVFLFFLSLTGTHC;
SEQ ID NO: 79: MEWSWVFLFFLSLTGTHT;
SEQ ID NO: 80: MEWSWVFLFFLSLTGTHQ;
SEQ ID NO: 81: MEWSWVFLFFLSLTGGHA;
SEQ ID NO: 82: MEWSWVFLFFLSLTGGHG;
SEQ ID NO: 83: MEWSWVFLFFLSLTGGHS;
SEQ ID NO: 84: MEWSWVFLFFLSLTGGHC;
SEQ ID NO: 85: MEWSWVFLFFLSLTGGHT;
SEQ ID NO: 86: MEWSWVFLFFLSLTGGHQ;
SEQ ID NO: 87: MEWSWVFLFFLSLTGCHA;
SEQ ID NO: 88: MEWSWVFLFFLSLTGCHG;
SEQ ID NO: 89: MEWSWVFLFFLSLTGCHS;
SEQ ID NO: 90: MEWSWVFLFFLSLTGCHC;
SEQ ID NO: 91: MEWSWVFLFFLSLTGCHT;
SEQ ID NO: 92: MEWSWVFLFFLSLTGCHQ;
SEQ ID NO: 93: MEWSWVFLFFLSLTGLHA;
SEQ ID NO: 94: MEWSWVFLFFLSLTGLHG;
SEQ ID NO: 95: MEWSWVFLFFLSLTGLHS;
SEQ ID NO: 96: MEWSWVFLFFLSLTGLHC;
SEQ ID NO: 97: MEWSWVFLFFLSLTGLHT;
SEQ ID NO: 98: MEWSWVFLFFLSLTGLHQ;
SEQ ID NO: 99: MEWSWVFLFFLSLTGIHA;
SEQ ID NO: 100: MEWSWVFLFFLSLTGIHG;
SEQ ID NO: 101: MEWSWVFLFFLSLTGIHS;
SEQ ID NO: 102: MEWSWVFLFFLSLTGIHC;
SEQ ID NO: 103: MEWSWVFLFFLSLTGIHT;
SEQ ID NO: 104: MEWSWVFLFFLSLTGIHQ;
SEQ ID NO: 105: MSVPTQVLGLLLLWLTDVRA;
SEQ ID NO: 106: MSVPTQVLGLLLLWLTDVRG;
SEQ ID NO: 107: MSVPTQVLGLLLLWLTDVRS;
SEQ ID NO: 108: MSVPTQVLGLLLLWLTDVRC;
SEQ ID NO: 109: MSVPTQVLGLLLLWLTDVRT;
SEQ ID NO: 110: MSVPTQVLGLLLLWLTDVRQ;
SEQ ID NO: 111: MSVPTQVLGLLLLWLTDARA;
SEQ ID NO: 112: MSVPTQVLGLLLLWLTDARG;
SEQ ID NO: 113: MSVPTQVLGLLLLWLTDARS;
SEQ ID NO: 114: MSVPTQVLGLLLLWLTDART;
SEQ ID NO: 115: MSVPTQVLGLLLLWLTDARQ;
SEQ ID NO: 116: MSVPTQVLGLLLLWLTDSRA;
SEQ ID NO: 117: MSVPTQVLGLLLLWLTDSRG;
SEQ ID NO: 118: MSVPTQVLGLLLLWLTDSRS;
SEQ ID NO: 119: MSVPTQVLGLLLLWLTDSRC;
SEQ ID NO: 120: MSVPTQVLGLLLLWLTDSRT;
SEQ ID NO: 121: MSVPTQVLGLLLLWLTDSRQ;
SEQ ID NO: 122: MSVPTQVLGLLLLWLTDTRA;
SEQ ID NO: 123: MSVPTQVLGLLLLWLTDTRG;
SEQ ID NO: 124: MSVPTQVLGLLLLWLTDTRS;
SEQ ID NO: 125: MSVPTQVLGLLLLWLTDTRC;
SEQ ID NO: 126: MSVPTQVLGLLLLWLTDTRT;
SEQ ID NO: 127: MSVPTQVLGLLLLWLTDTRQ;
SEQ ID NO: 128: MSVPTQVLGLLLLWLTDGRA;
SEQ ID NO: 129: MSVPTQVLGLLLLWLTDGRG;
SEQ ID NO: 130: MSVPTQVLGLLLLWLTDGRS;
SEQ ID NO: 131: MSVPTQVLGLLLLWLTDGRC;
SEQ ID NO: 132: MSVPTQVLGLLLLWLTDGRT;
SEQ ID NO: 133: MSVPTQVLGLLLLWLTDGRQ;
SEQ ID NO: 134: MSVPTQVLGLLLLWLTDCRA;
SEQ ID NO: 135: MSVPTQVLGLLLLWLTDCRG;
SEQ ID NO: 136: MSVPTQVLGLLLLWLTDCRS;
SEQ ID NO: 137: MSVPTQVLGLLLLWLTDCRC;
SEQ ID NO: 138: MSVPTQVLGLLLLWLTDCRT;
SEQ ID NO: 139: MSVPTQVLGLLLLWLTDCRQ;
SEQ ID NO: 140: MSVPTQVLGLLLLWLTDLRA;
SEQ ID NO: 141: MSVPTQVLGLLLLWLTDLRG;
SEQ ID NO: 142: MSVPTQVLGLLLLWLTDLRS;
SEQ ID NO: 143: MSVPTQVLGLLLLWLTDLRC;
SEQ ID NO: 144: MSVPTQVLGLLLLWLTDLRT;
SEQ ID NO: 145: MSVPTQVLGLLLLWLTDLRQ;
SEQ ID NO: 146: MSVPTQVLGLLLLWLTDIRA;
SEQ ID NO: 147: MSVPTQVLGLLLLWLTDIRG;
SEQ ID NO: 148: MSVPTQVLGLLLLWLTDIRS;
SEQ ID NO: 149: MSVPTQVLGLLLLWLTDIRC;
SEQ ID NO: 150: MSVPTQVLGLLLLWLTDIRT; and
SEQ ID NO: 151: MSVPTQVLGLLLLWLTDIRQ.

In some embodiments, provided is the method for reducing N-terminal heterogeneity of a heavy chain of an antibody and/or a light chain of an antibody described above, wherein the first signal peptide or the second signal peptide each independently comprises an amino acid sequence of SEQ ID NO: 57, SEQ ID NO: 58, SEQ ID NO: 59, SEQ ID NO: 93, SEQ ID NO: 94, SEQ ID NO: 95, SEQ ID NO: 99, SEQ ID NO: 100, SEQ ID NO: 101, SEQ ID NO: 105, SEQ ID NO: 106, SEQ ID NO: 107, SEQ ID NO: 140, SEQ ID NO: 141, SEQ ID NO: 142, SEQ ID NO: 146, SEQ ID NO: 147 or SEQ ID NO: 148.

In some embodiments, provided is the method for reducing N-terminal heterogeneity of a heavy chain of an antibody and/or a light chain of an antibody described above, wherein the first signal peptide comprises the amino acid sequence of SEQ ID NO: 57 or SEQ ID NO: 105, and/or the second signal peptide comprises the amino acid sequence of SEQ ID NO: 57 or SEQ ID NO: 105.

In some embodiments, provided is the method for reducing N-terminal heterogeneity of a heavy chain of an antibody and/or a light chain of an antibody described above, wherein the first signal peptide comprises the amino acid sequence of SEQ ID NO: 57, and/or the second signal peptide comprises the amino acid sequence of SEQ ID NO: 57. In some embodiments, provided is the method for reducing N-terminal heterogeneity of a heavy chain of an antibody and/or a light chain of an antibody described above, wherein the first signal peptide comprises the amino acid sequence of SEQ ID NO: 57, and/or the second signal peptide comprises the amino acid sequence of SEQ ID NO: 105.

In some embodiments, provided is the method for reducing N-terminal heterogeneity of a heavy chain of an antibody and/or a light chain of an antibody described above, wherein the first signal peptide comprises the amino acid sequence of SEQ ID NO: 105, and/or the second signal peptide comprises the amino acid sequence of SEQ ID NO: 57. In some embodiments, provided is the method for reducing N-terminal heterogeneity of a heavy chain of an antibody and/or a light chain of an antibody described above, wherein the first signal peptide comprises the amino acid sequence of SEQ ID NO: 105, and/or the second signal peptide comprises the amino acid sequence of SEQ ID NO: 105.

In some embodiments, provided is the method for reducing N-terminal heterogeneity of a heavy chain of an antibody and/or a light chain of an antibody described above, wherein the first polynucleotide or the second polynucleotide each independently encodes a polypeptide comprising an amino acid sequence selected from the group consisting of SEQ ID NO: 157, SEQ ID NO: 158, SEQ ID NO: 159, SEQ ID NO: 160, SEQ ID NO: 163, SEQ ID NO: 164, SEQ ID NO: 165, SEQ ID NO: 166, SEQ ID NO: 171 and SEQ ID NO: 172.

In some embodiments, provided is the method for reducing N-terminal heterogeneity of a heavy chain of an antibody and/or a light chain of an antibody described above, wherein the host cell is a eukaryotic host cell; in some embodiments, the eukaryotic host cell is a CHO cell or a yeast.

In some embodiments, provided is the method for reducing N-terminal heterogeneity of a heavy chain of an antibody and/or a light chain of an antibody described above, wherein the antibody is a murine antibody, a chimeric antibody, a humanized antibody, a human antibody, an affinity-matured antibody or a multispecific antibody.

In some embodiments, provided is the method for reducing N-terminal heterogeneity of a heavy chain of an antibody and/or a light chain of an antibody described above, wherein the antibody is selected from the group consisting of an anti-TLR7 antibody, an anti-HER2 (ErbB2) antibody, an anti-Claudin18.2 antibody, an anti-EGFR antibody, an anti-B7H3 antibody, an anti-c-Met antibody, an anti-HER3 (ErbB3) antibody, an anti-HER4 (ErbB4) antibody, an anti-CD3 antibody, an anti-CD20 antibody, an anti-CD22 antibody, an anti-CD30 antibody, an anti-CD33 antibody, an anti-CD38 antibody, an anti-CD44 antibody, an anti-CD47 antibody, an anti-CD56 antibody, an anti-CD70 antibody, an anti-CD73 antibody, an anti-CD105 antibody, an anti-CEA antibody, an anti-A33 antibody, an anti-Cripto antibody, an anti-SOST antibody, an anti-EphA2 antibody, an anti-G250 antibody, an anti-MUCl antibody, an anti-Lewis Y antibody, an anti-VEGFR antibody, an anti-GPNMB antibody, an anti-thrombin antibody, an anti-Aβ antibody, an anti-Integrin antibody, an anti-ANGPTL3 antibody, an anti-PSMA antibody, an anti-PD-1 antibody, an anti-PD-L1 antibody, an anti-LAG3 antibody, an anti-IL-5 antibody, an anti-IL-15 antibody, an anti-IL-4R antibody, an anti-IL-6R antibody, an anti-TIGHT antibody, an anti-Tenascin-C antibody, an anti-SLC44A4 antibody, an anti-PCSK9 antibody, an anti-EpCAM antibody, an anti-CTGF antibody, an anti-TSLP antibody, an anti-CEA antibody, an anti-Mesothelin antibody and an anti-FcRn antibody.

In some embodiments, provided is the method for reducing N-terminal heterogeneity of a heavy chain of an antibody and/or a light chain of an antibody described above, wherein the antibody is selected from the group consisting of trastuzumab, pertuzumab, nimotuzumab, enoblituzumab, emibetuzumab, inotuzumab, pinatuzumab, brentuximab, gemtuzumab, bivatuzumab, lorvotuzumab, cBR96, glematumamab, an anti-Claudin18.2 antibody and an anti-FcRn antibody, wherein a heavy chain and a light chain of the anti-Claudin18.2 antibody comprise amino acid sequences of SEQ ID NO: 49 and SEQ ID NO: 47, respectively; a heavy chain and a light chain of the anti-FcRn antibody comprise amino acid sequences of SEQ ID NO: 167 and SEQ ID NO: 168, respectively. In some embodiments, provided is the method for reducing N-terminal heterogeneity of a heavy chain of an antibody and/or a light chain of an antibody described above, which comprises cloning a light chain plasmid into a heavy chain plasmid to construct a full-length antibody plasmid. In some embodiments, in the method, the plasmid is introduced into the host cell by electroporation.

The present disclosure further provides a signal peptide comprising or consisting of an amino acid sequence of SEQ ID NO: 55 or SEQ ID NO: 56. In some embodiments, the polypeptide is a signal peptide having an amino acid sequence set forth in any one of SEQ ID NO: 55 to SEQ ID NO: 151.

Provided is a polypeptide, which comprises a signal peptide comprising an amino acid sequence of SEQ ID NO: 55 or SEQ ID NO: 56.

In some embodiments, provide is the polypeptide described above, wherein the polypeptide further comprises a heterologous polypeptide operably linked to the signal peptide; in some embodiments, the heterologous polypeptide is a heavy chain of an antibody or a light chain of an antibody; in some embodiments, the signal peptide is operably linked to the N-terminus of the heavy chain of the antibody or the light chain of the antibody.

In some embodiments, provided is the polypeptide described above, wherein the signal peptide comprises an amino acid sequence selected from the group consisting of SEQ ID NO: 57 to SEQ ID NO: 151.

In some embodiments, provided is the polypeptide described above, wherein the signal peptide comprises an amino acid sequence of SEQ ID NO: 57, SEQ ID NO: 58, SEQ ID NO: 59, SEQ ID NO: 93, SEQ ID NO: 94, SEQ ID NO: 95, SEQ ID NO: 99, SEQ ID NO: 100, SEQ ID NO: 101, SEQ ID NO: 105, SEQ ID NO: 106, SEQ ID NO: 107, SEQ ID NO: 140, SEQ ID NO: 141, SEQ ID NO: 142, SEQ ID NO: 146, SEQ ID NO: 147 or SEQ ID NO: 148.

In some embodiments, provided is the polypeptide described above, wherein the signal peptide comprises an amino acid sequence selected from the group consisting of SEQ ID NO: 57 and SEQ ID NO: 105.

In some embodiments, provided is the polypeptide described above, wherein the polypeptide comprises an amino acid sequence selected from the group consisting of SEQ ID NO: 157, SEQ ID NO: 158, SEQ ID NO: 159, SEQ ID NO: 160, SEQ ID NO: 163, SEQ ID NO: 164, SEQ ID NO: 165, SEQ ID NO: 166, SEQ ID NO: 169, SEQ ID NO: 170, SEQ ID NO: 171 and SEQ ID NO: 172.

The present disclosure further provides a nucleic acid molecule, which encodes the polypeptide according to any one of the preceding embodiments.

The present disclosure further provides a host cell, which comprises the nucleic acid molecule described above.

In some embodiments, provided is the host cell described above, wherein the host cell is a eukaryotic host cell; in some embodiments, the eukaryotic host cell is a CHO cell or a yeast.

The present disclosure further provides a composition, which comprises a heterologous polypeptide having a terminal extension ratio of less than 3%, 2.5%, 2%, 1.5%, or 1%; in some embodiments, the heterologous polypeptide obtained through expression has a terminal extension ratio of less than 1%; in some embodiments, the heterologous polypeptide obtained through expression has a terminal extension ratio of less than 1%, and the terminal extension ratio is measured based on a peptide mapping assay; in some embodiments, the heterologous polypeptide obtained through expression is substantially free of residual terminal residues; in some embodiments, the heterologous polypeptide obtained through expression has a terminal extension ratio of 0%.

In some embodiments, provided is the composition described above, wherein the heterologous polypeptide is a heavy chain of an antibody and/or a light chain of an antibody; in some embodiments, the antibody is selected from the group consisting of trastuzumab, pertuzumab, an anti-Claudin18.2 antibody and an anti-FcRn antibody, wherein a heavy chain and a light chain of the anti-Claudin18.2 antibody comprise amino acid sequences of SEQ ID NO: 49 and SEQ ID NO: 47, respectively.

In some embodiments, the antibody is selected from an anti-FcRn antibody, wherein a heavy chain and a light chain of the anti-FcRn antibody comprise amino acid sequences of SEQ ID NO: 167 and SEQ ID NO: 168, respectively.

In some embodiments, provided is the method for reducing N-terminal heterogeneity of a heavy chain of an antibody and/or a light chain of an antibody described above, wherein the heavy chain of the antibody and/or the light chain of an antibody obtained through expression have a terminal extension ratio of less than 3%, 2.5%, 2%, 1.5%, or 1%; in some embodiments, the heavy chain of the antibody and/or the light chain of the antibody obtained through expression have a terminal extension ratio of less than 1%; in some embodiments, the heavy chain of the antibody and/or the light chain of the antibody obtained through expression have a terminal extension ratio of less than 1%, and the terminal extension ratio is measured based on a peptide mapping assay; in some embodiments, the heavy chain of the antibody and/or the light chain of the antibody obtained through expression are substantially free of residual terminal residues; in some embodiments, the heavy chain of the antibody and/or the light chain of the antibody obtained through expression have a terminal extension ratio of 0%.

In some embodiments, provided is the composition described above, which is prepared by the method according to any one of the preceding embodiments.

The signal peptide provided by the present disclosure has the effect of stably reducing terminal heterogeneity of a heterologous polypeptide, and is suitable for expressing heterologous polypeptides in large scale.

### BRIEF DESCRIPTION OF THE DRAWINGS

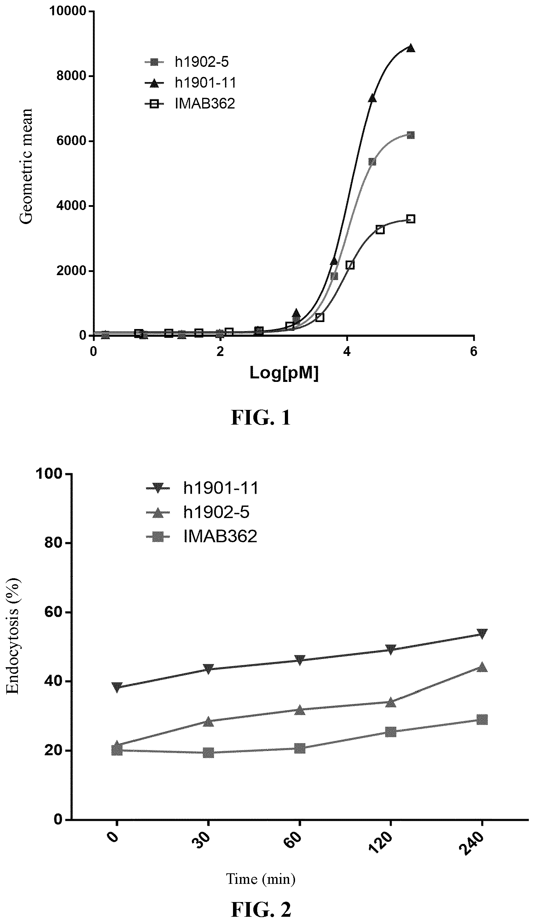

FIG. 1: the results of FACS detection of the binding of humanized antibodies to human Claudin18.2 at cell level.
FIG. 2: endocytosis of humanized antibodies by NUGC4 cells.

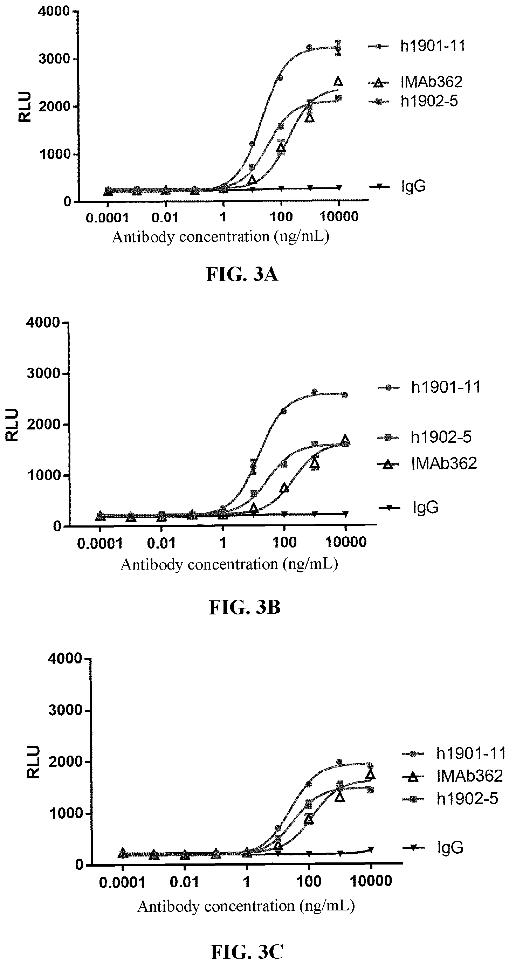

FIGs. 3A-3C: the detection of ADCC effect of antibodies in NUGC4 cells with varying expression levels of Claudin18.2; FIG. 3A shows the detection of ADCC effect of antibodies in wild-type NUGC4 cells (with low Claudin18.2 expression); FIG. 3B shows the detection of ADCC effect of antibodies in NUGC4 cells with moderate Claudin18.2 expression; FIG. 3C shows the detection of ADCC effect of antibodies in NUGC4 cells with high Claudin18.2 expression.

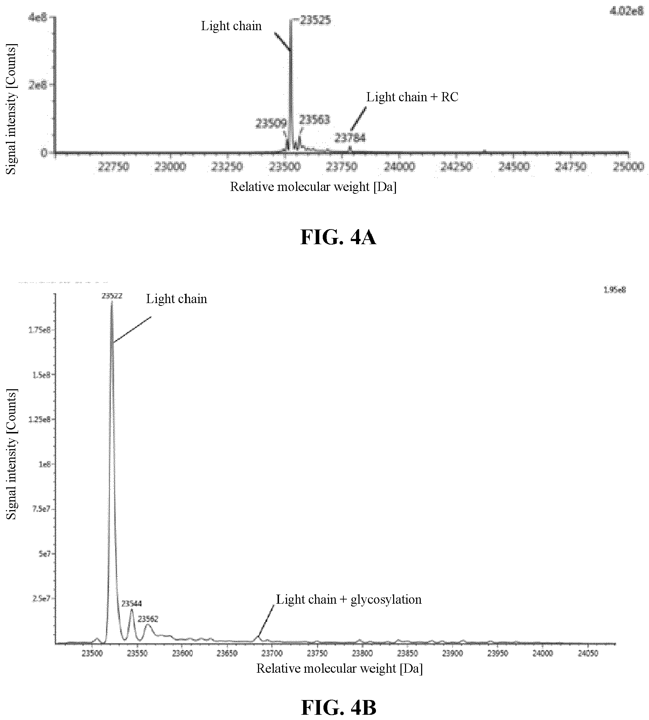

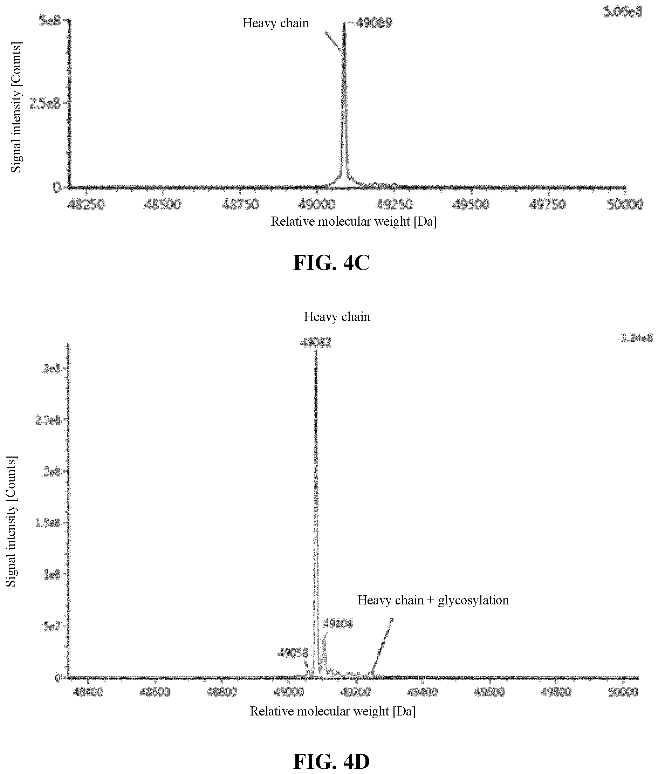

FIGs. 4A-4D: FIG. 4A shows the mass spectrum of the molecular weight after deglycosylation and reduction of the light chain of pertuzumab-1 antibody; FIG. 4B shows the mass spectrum of the molecular weight after deglycosylation and reduction of the light chain of pertuzumab-3 antibody; FIG. 4C shows the mass spectrum of the molecular weight after deglycosylation and reduction of the heavy chain of pertuzumab-1 antibody; FIG. 4D shows the mass spectrum of the molecular weight after deglycosylation and reduction of the heavy chain of pertuzumab-3 antibody.

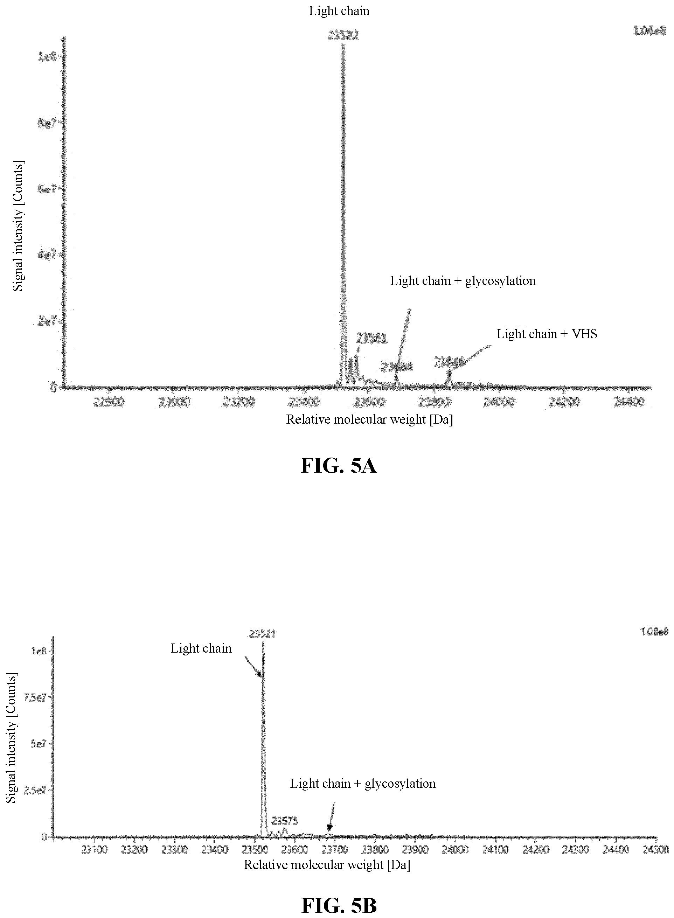

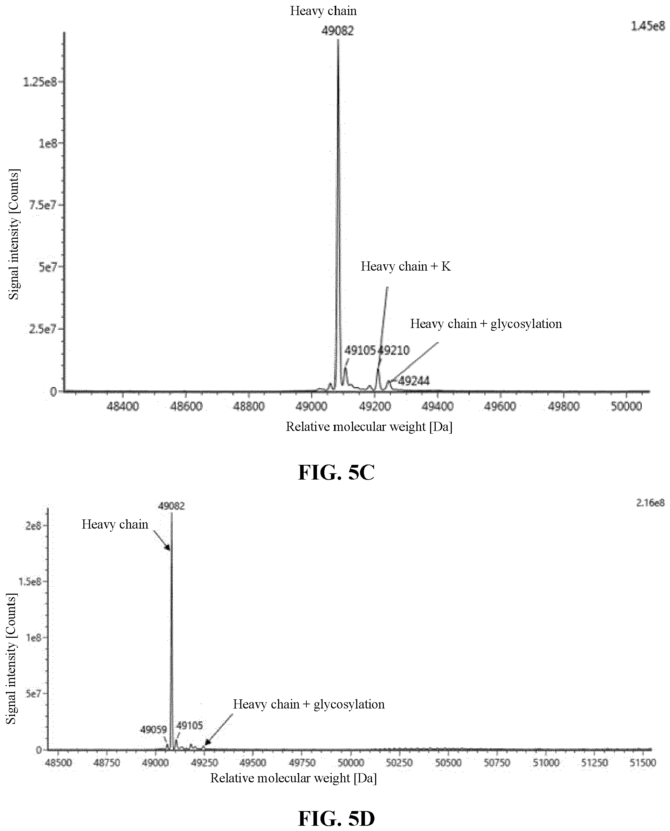

FIGs. 5A-5D: FIG. 5A shows the mass spectrum of the molecular weight after deglycosylation and reduction of the light chain of pertuzumab-2 antibody; FIG. 5B shows the mass spectrum of the molecular weight after deglycosylation and reduction of the light chain of pertuzumab-4 antibody; FIG. 5C shows the mass spectrum of the molecular weight after deglycosylation and reduction of the heavy chain of pertuzumab-2 antibody; FIG. 5D shows the mass spectrum of the molecular weight after deglycosylation and reduction of the heavy chain of pertuzumab-4 antibody.

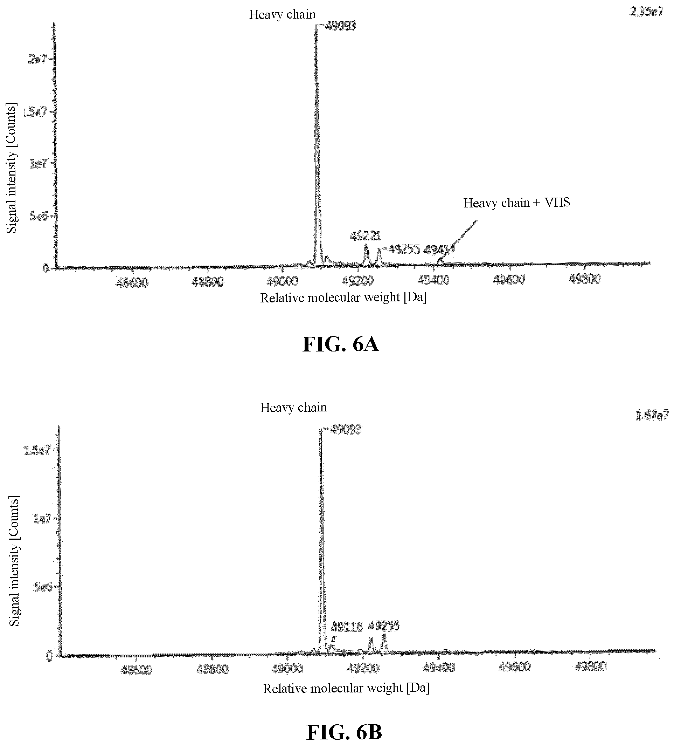

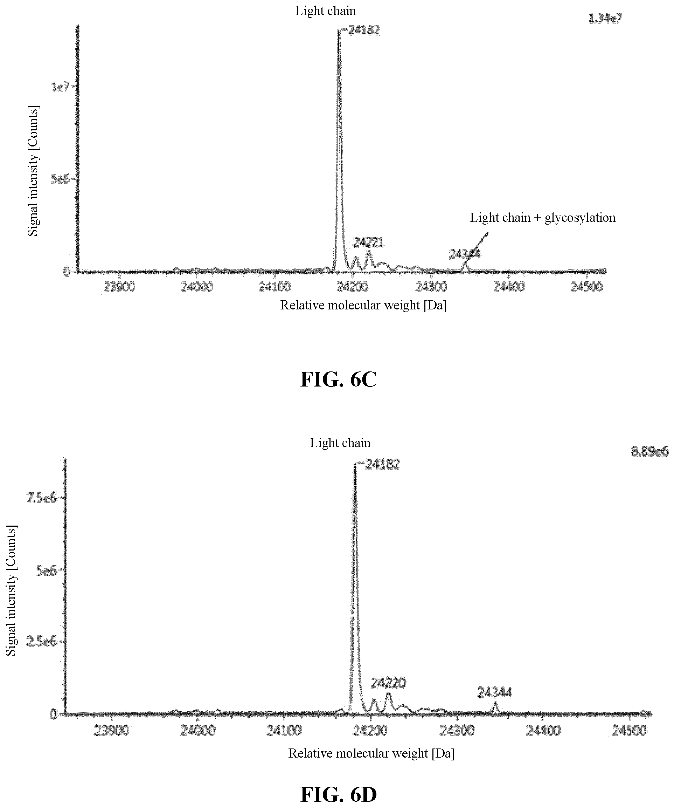

FIGs. 6A-6D: FIG. 6A shows the mass spectrum of the molecular weight after deglycosylation and reduction of the light chain of h1902-5-1 antibody; FIG. 6B shows the mass spectrum of the molecular weight after deglycosylation and reduction of the light chain of h1902-5-2 antibody; FIG. 6C shows the mass spectrum of the molecular weight after deglycosylation and reduction of the heavy chain of h1902-5-1 antibody;
FIG. 6D shows the mass spectrum of the molecular weight after deglycosylation and reduction of the heavy chain of h1902-5-2 antibody.

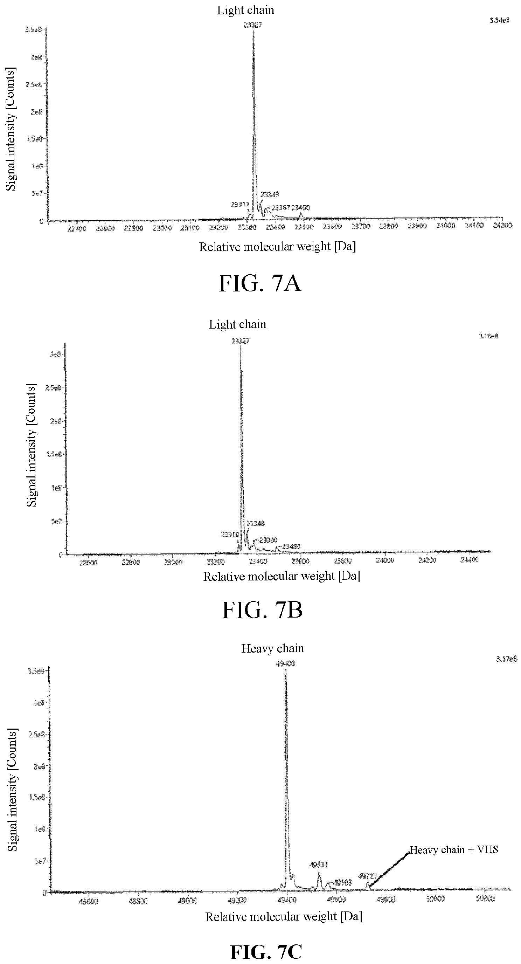

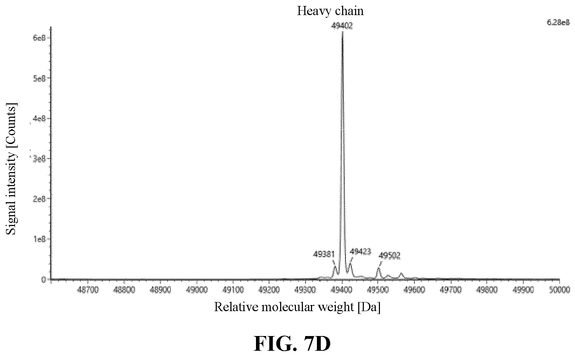

FIGs. 7A-7D: FIG. 7A shows the mass spectrum of the molecular weight after deglycosylation and reduction of the light chain of FcRn-1 antibody; FIG. 7B shows the mass spectrum of the molecular weight after deglycosylation and reduction of the light chain of FcRn-2 antibody; FIG. 7C shows the mass spectrum of the molecular weight after deglycosylation and reduction of the heavy chain of FcRn-1 antibody; FIG. 7D shows the mass spectrum of the molecular weight after deglycosylation and reduction of the heavy chain of FcRn-2 antibody.

### DETAILED DESCRIPTION

### Detailed Description of the Invention

### Terms

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure belongs.

The three-letter and single-letter codes for amino acids used herein are as described in J. biol. chem, 243, p3558 (1968).

The term "antibody" described herein is used in the broadest sense herein and includes different antibody structures including, but not limited to, a monoclonal antibody, a polyclonal antibody, a murine antibody, a chimeric antibody, a humanized antibody, a multispecific antibody (e.g., a bispecific antibody) and antibody fragments, so long as they exhibit the desired antigen-binding activity and specificity.

The term "antibody fragment" refers to a molecule different from an intact antibody, and it comprises a portion of an intact antibody that specifically binds to an antigen to which the intact antibody specifically binds. Examples of antibody fragments include, but are not limited to, Fv, Fab, Fab', Fab'-SH, F(ab')₂, diabodies, linear antibodies, single-chain antibody molecules (e.g., scFv or scFab), single-domain antibodies (dAbs), and multispecific antibodies formed from antibody fragments.

The term "chimeric antibody" described herein refers to an antibody obtained by fusing a variable region of a murine antibody and a constant region of a human antibody, which can reduce an immune response induced by the murine antibody.

The term "humanized antibody" described herein, also known as a CDR-grafted antibody, refers to an antibody produced by grafting murine CDR sequences into a human antibody variable region framework, i.e., a different type of human germline antibody framework sequence. Such an antibody can overcome the heterogeneous reaction induced by the chimeric antibody because of carrying a large amount of mouse protein component. To avoid the decrease in activity caused by the decrease in immunogenicity, the framework region sequence in human antibody variable region can be subjected to minimum reverse mutation or back mutation to maintain or enhance activity. The humanized antibody of the present disclosure also includes humanized antibodies that were further subjected to CDR affinity maturation mutation by yeast display.

The terms "human antibody" (HuMAb), "human-derived antibody", "fully human antibody" and "completely human antibody" herein are used interchangeably and have amino acid sequences corresponding to those of antibodies produced by humans or human cells, or derived from non-human sources using repertoires of human antibodies or other human antibody coding sequences. The definition of such a human antibody specifically excludes humanized antibodies comprising non-human antigen-binding residues.

The term "antibody fragment" described herein refers to a molecule different from an intact antibody, and it comprises a portion of an intact antibody that specifically binds to an antigen to which the intact antibody specifically binds. Examples of antibody fragments include, but are not limited to, Fv, Fab, Fab', Fab'-SH, F(ab')₂, diabodies, linear antibodies, single-chain antibody molecules (e.g., scFv or scFab), single-domain antibodies (dAbs), and multispecific antibodies formed from antibody fragments. The term "antibody" of the present disclosure includes "intact antibodies" and "antibody fragments" thereof. Accordingly, the heavy chain of an antibody or the light chain of an antibody described herein includes a complete heavy chain or a complete light chain, and also includes a heavy chain fragment or a light chain fragment of an antibody fragment.

The term "framework" or "FR" refers to variable domain residues other than complementarity determining region (CDR) residues. The FR of a variable domain generally consists of four FR domains: FR1, FR2, FR3 and FR4. Thus, CDR and FR sequences typically occur in a VH (or VL) in the following order:

FR1-HCDR1(LCDR1)-FR2-HCDR2(LCDR2)-FR3-HCDR3(LCDR3)-FR4.

The term "complementarity determining region", "CDR" or "hypervariable region" refers to one of the 6 hypervariable regions within variable domains of an antibody, which primarily contribute to antigen binding. Generally, there are three CDRs (HCDR1, HCDR2 and HCDR3) in each heavy chain variable region and three CDRs (LCDR1, LCDR2 and LCDR3) in each light chain variable region. The amino acid sequence boundaries of the CDRs can be determined using any one of a variety of well-known schemes, including "Kabat" numbering scheme (see Kabat et al. (1991), "Sequences of Proteins of Immunological Interest", 5th edition, Public Health Service, National Institutes of Health, Bethesda, MD), "Chothia" numbering scheme (see Al-Lazikani et al. (1997) JMB 273: 927-948) and ImMunoGenTics (IMGT) numbering scheme (see Lefranc M.P., Immunologist, 7, 132-136 (1999); Lefranc, M.P. et al., Dev. Comp. Immunol., 27, 55-77 (2003)), and the like. For example, for the classical format, according to the Kabat scheme, the CDR amino acid residues in the heavy chain variable domain (VH) are numbered 31-35 (HCDR1), 50-65 (HCDR2) and 95-102 (HCDR3); the CDR amino acid residues in the light chain variable domain (VL) are numbered 24-34 (LCDR1), 50-56 (LCDR2) and 89-97 (LCDR3). According to the Chothia scheme, the CDR amino acids in VH are numbered 26-32 (HCDR1), 52-56 (HCDR2) and 95-102 (HCDR3); and amino acid residues in VL are numbered 26-32 (LCDR1), 50-52 (LCDR2) and 91-96 (LCDR3). According to the CDR definitions of both the Kabat scheme and the Chothia scheme, the CDR is composed of amino acid residues 26-35 (HCDR1), 50-65 (HCDR2) and 95-102 (HCDR3) in the human VH and amino acid residues 24-34 (LCDR1), 50-56 (LCDR2) and 89-97 (LCDR3) in the human VL. According to the IMGT scheme, the CDR amino acid residues in VH are roughly numbered 26-35 (CDR1), 51-57 (CDR2) and 93-102 (CDR3), and the CDR amino acid residues in VL are roughly numbered 27-32 (CDR1), 50-52 (CDR2) and 89-97 (CDR3). According to the IMGT scheme, the CDRs of the antibody can be determined using the program IMGT/DomainGap Align. Unless otherwise stated, the "Kabat" numbering scheme is applied to the sequences of the variable regions and CDRs of the antibody involved in examples of the present disclosure.

The terms "specific binding", "selective binding", "selectively bind to" and "specifically bind to" refer to the binding of an antibody to an epitope on a predetermined antigen. Generally, the antibody binds with an affinity (KD) of less than about 10⁻⁸ M, e.g., less than about 10⁻⁹ M, 10⁻¹¹ M, 10⁻¹¹ M, 10⁻¹² M, or less.

The term "KD" refers to the dissociation equilibrium constant for specific antibody-antigen interaction. Typically, an antibody of the present disclosure binds to an antigen with a dissociation equilibrium constant (KD) of less than about 10⁻⁷ M, e.g., less than about 10⁻⁸ M or 10⁻⁹ M. For example, the affinity (KD value) of an antibody to an antigen on the cell surface in the present disclosure is determined by FACS method.

The term "polynucleotide" or "nucleic acid molecule" used interchangeably in the present disclosure refers to a polymer of nucleotides of any length, and includes DNA and RNA. The nucleotides may be deoxyribonucleotides, ribonucleotides, modified nucleotides or bases, and/or analogs thereof, or any substrates that can be incorporated into a polymer by a DNA or RNA polymerase or by a synthetic reaction. Polynucleotides may include modified nucleotides, such as methylated nucleotides and analogs thereof. If present, modifications to the structure of the nucleotide can be made before or after the assembly of the polymer. The sequence of a nucleotide can be interrupted by non-nucleotide components.

The term "operably linked" described herein refers to the juxtaposition of two or more components, wherein the components are in a relationship that allows them to function in their intended manner. For example, a promoter is operably linked to a coding sequence if it acts in cis to control or regulate the transcription of the linked sequence. Generally, but not necessarily, "operably linked" DNA sequences are contiguous and, where necessary to link two protein coding regions or where a secretory leader sequence is involved, contiguous and in reading frame. However, while an operably linked promoter is typically located upstream of a coding sequence, it is not necessarily contiguous therewith. An operably linked enhancer may be located upstream of, within or downstream of a coding sequence and at a considerable distance from the promoter. Linking is accomplished by recombinant methods known in the art, e.g., using PCR method, by annealing, or by linking at convenient restriction sites. If convenient restriction sites do not exist, synthetic oligonucleotide adaptors or linkers are used according to conventional practice. In the present disclosure, one way of linking a signal peptide to a heterologous polypeptide is direct linking.

The term "host cell" described herein refers to a cell that has been genetically altered or is capable of being genetically altered by the introduction of an exogenous polynucleotide (such as a recombinant plasmid or vector). It will be understood that such terms are intended to refer not only to the particular individual cell, but also to the progeny of such a cell. Because certain modifications may occur in successive generations due to either mutation or environmental influence, such progeny may not, in fact, be identical to the parent cell, but are still included within the scope of the term "host cell" used herein. The host cell may be a microorganism (e.g., a eukaryotic microorganism) or an animal cell. Suitable microorganisms include *Saccharomyces cerevisiae* and *Pichia pastoris,* and suitable animal host cell lines include CHO (Chinese hamster ovary cell line), 293 cells and NS0 cells.

The term "polypeptide" described herein broadly refers to a peptide and a protein derived from any cellular source and having more than about 10 amino acids. "Heterologous" polypeptides are those that are foreign to the host cell utilized, such as human proteins produced by the host cell. Heterologous polypeptides may be prokaryotic or eukaryotic, such as mammalian or human. A heterologous polypeptide may be a recombinantly produced polypeptide or a recombinant polypeptide.

Exemplary heterologous polypeptides include transmembrane molecules (e.g., receptors, such as receptor tyrosine kinases) or ligands such as growth factors. Exemplary heterologous polypeptides include molecules such as: renin; growth hormones, including human growth hormone and bovine growth hormone; growth hormone releasing factor; parathyroid hormone; thyroid stimulating hormone; lipoproteins; α1-antitrypsin; insulin A-chain; insulin B-chain; proinsulin; follicle stimulating hormone; calcitonin; luteinizing hormone; glucagon; clotting factors, such as factor VIIIC, factor IX, tissue factor (TF) and von Willebrands factor; anti-clotting factors, such as protein C; atrial natriuretic peptide; lung surfactant; plasminogen activator, such as urokinase or human urine or tissue-type plasminogen activator (t-PA); bombesin; thrombin; hemopoietic growth factor; tumor necrosis factor-α and -β; enkephalinase; RANTES (regulated on activation normally T-cell expressed and secreted); human macrophage inflammatory protein (MIP-1-α); serum albumin, such as human serum albumin; Muellerian-inhibiting substance; relaxin A-chain; relaxin B-chain; prorelaxin; mouse gonadotropin-associated peptide; microbial protein, such as β-lactamase; DNase; IgE; cytotoxic T-lymphocyte associated antigen (CTLA), such as CTLA-4; inhibin; activin; vascular endothelial growth factor (VEGF); receptors for hormones or growth factors; protein A or D; rheumatoid factors; neurotrophic factors, such as bone-derived neurotrophic factor (BDNF), neurotrophin-3, -4, -5 or -6 (NT-3, NT-4, NT-5 or NT-6), or nerve growth factors, such as NGFβ; platelet-derived growth factor (PDGF); fibroblast growth factor, such as aFGF and bFGF; epidermal growth factor (EGF); transforming growth factor (TGF), such as TGF-α and TGF-β, including TGF-β1, TGF-β2, TGF-β3, TGF-β4 or TGF-β5; insulin-like growth factor-I and -II (IGF-I and IGF-II); des(1-3)-IGF-I (brain IGF-I), insulin-like growth factor binding proteins; CD proteins, such as CD3, CD4, CD8, CD19, CD20 and CD40; erythropoietin; osteoinductive factors; immunotoxins; bone morphogenetic protein (BMP); interferons, such as interferon-α, -β, and -γ; colony stimulating factor (CSF), such as M-CSF, GM-CSF and G-CSF; interleukin (IL), such as IL-1 to IL-10; superoxide dismutase; T-cell receptors; surface membrane proteins; decay accelerating factor; viral antigen, such as a portion of the AIDS envelope; transport proteins; homing receptors; addressin; regulatory proteins; integrins, such as CD11a, CD11b, CD11c, CD18, ICAM, VLA-4 and VCAM; tumor associated antigens, such as HER2, HER3 or HER4 receptor; and fragments of any of the above polypeptides.

A heterologous polypeptide may also be an antibody, and illustratively, targets of the antibody include, but are not limited to A33, BMPI, BMP2, BMP3B (GDFIO), BMP4, BMP6, BMP8, CSFI (M-CSF), CSF2 (GM-CSF), CSF3 (G-CSF), EPO, FGFI (aFGF), FGF2 (bFGF), FGF3 (int-2), FGF4 (HST), FGF5, FGF6 (HST-2), FGF7 (KGF), FGF9, FGF10, FGF11, FGF12, FGF12B, FGF14, FGF16, FGF17, FGF19, FGF20, FGF21, FGF23, IGF1, IGF2, IFNAI, IFNA2, IFNA4, IFNA5, IFNA6, IFNA7, IFNBI, IFNG, IFNWI, FELI, FELI (EPSELON), FELI (ZETA), IL1A, IL1B, IL2, IL3, IL4, IL5, IL6, IL7, IL8, IL9, IL10, IL11, IL12A, I L12B, IL13, IL14, IL15, I L16, IL17, IL17B, IL18, IL19, IL20, IL22, IL23, IL24, IL25, IL26, IL27, IL28A, IL28B, I L29, IL30, PDGFA, PDGFB, TGFA, TGFB1, TGFB2, TGFB3, LTA(TNF-b), LTB, TNF (TNF-a), TNFSF4 (OX40 ligand), TNFSF5 (CD40 ligand), TNFSF6 (FasL), TNFSF7 (CD27 ligand), TNFSF8 (CD30 ligand), TNFSF9 (4-1BB ligand), TNFSFIO (TRAIL), TNFSF1I (TRANCE), TNFSF12 (AP03L), TNFSF13 (April), TNFSF13B, TNFSF14 (HVEM-L), TNFSF15 (VEGI), TNFSF18, HGF (VEGFD), VEGF, VEGFB, VEGFC, ILIR1, IL1R2, IL1RL1, IL1RL2, IL2RA, IL2RB, IL2RG, IL3RA, IL4R, IL5RA, IL6R, IL7R, IL8RA, IL8RB, IL9R, ILIORA, ILIORB, IL11RA, IL12RB1, IL12RB2, IL13RA1, IL13RA2, IL15RA, IL17R, IL18R1, IL20RA, IL21R, IL22R, IL1HY1, IL1RAP, IL1RAPL1, IL1RAPL2, IL1RN, IL6ST, IL18BP, IL18RAP, IL22RA2, AIFI, HGF, LEP (leptin), PTN, THPO, CCLI (I-309), CCL2 (MCP-1/MCAF), CCL3 (MIP-la), CCL4 (MIP-lb), CCL5 (RANTES), CCL7 (MCP-3), CCL8 (mcp-2), CCLH (eotaxin), CCL13 (MCP-4), CCL15 (MIP-ld), CCL16 (HCC-4), CCL17 (TARC), CCL18 (PARC), CCL19 (MDP-3b), CCL20 (MIP-3a), CCL21 (SLC/exodus-2), CCL22 (MDC/STC-I), CCL23 (MPIF-I), CCL24 (MPIF-2/eotaxin-2), CCL25 (TECK), CCL26 (eotaxin-3), CCL27 (CTACK/ILC), CCL28, CXCLI (GROI), CXCL2 (GR02), CXCL3 (GR03), CXCL5 (ENA-78), CXCL6 (GCP-2), CXCL9 (MIG), CXCL10 (IP 10), CXCL11 (I-TAC), CXCL12 (SDFI), CXCL13, CXCL14, CXCL16, PF4 (CXCL4), PPBP (CXCL7), CX3CL1 (SCYDI), SCYEI, XCLI (lymphotactin), XCL2 (SCM-lb), BLRI (MDR15), CCBP2 (D6/JAB61), CCR1 (CKRI/HM145), CCR2 (mcp-IRB/RA), CCR3 (CKR3/CMKBR3), CCR4, CCR5 (CMKBR5/ChemR13), CCR6 (CMKBR6/CKR-L3/STRL22/DRY6), CCR7 (CKR7/EBII), CCR8 (CMKBR8/TERI/CKR-LI), CCR9 (GPR-9-6), CCRLI (VSHKI), CCRL2 (L-CCR), XCRI (GPR5/CCXCRI), CMKLRI, CMKORI (RDCI), CX3CR1 (V28), CXCR4, GPR2 (CCRIO), GPR31, GPR81 (FKSG80), CXCR3 (GPR9/CKR-L2), CXCR6 (TYMSTR/STRL33/Bonzo), HM74, IL8RA (IL8Ra), IL8RB (IL8Rb), LTB4R (GPR16), TCPIO, CKLFSF2, CKLFSF3, CKLFSF4, CKLFSF5, CKLFSF6, CKLFSF7, CKLFSF8, BDNF, C5R1, CSF3, GRCCIO (CIO), EPO, FY (DARC), GDF5, HDFIA, DL8, PRL, RGS3, RGS13, SDF2, SLIT2, TLR2, TLR4, TREMI, TREM2, VHL, ABCFI, ACVRI, ACVRIB, ACVR2, ACVR2B, ACVRLI, AD0RA2A, Aggrecan, AGR2, AICDA, AIFI, AIGI, AKAPI, AKAP2, AMH, AMHR2, ANGPTI, ANGPT2, ANGPTL3, ANGPTL4, ANPEP, APC, APOCI, AR, AZGPI (zinc-a-glycoprotein), Aβ, B7.1, B7.2, B7H3, BAD, BAFF (BLys), BAGI, BAH, BCL2, BCL6, BDNF, BLNK, BLRI (MDR15), BMPI, BMP2, BMP3B(GDFIO), BMP4, BMP6, BMP8, BMPRIA, BMPRIB, BMPR2, BPAGI (plectin), BRCAI, C19orflO (IL27w), C3, C4A, C5, C5R1, CANTI, CASP1, CASP4, CAVI, CCBP2 (D6/JAB61), CCLI (1-309), CCLII (eotaxin), CCL13 (MCP-4), CCL15 (MlP-ld), CCL16 (HCC-4), CCL17 (TARC), CCL18 (PARC), CCL19 (MIP-3b), CCL2 (MCP-1), MCAF, CCL20 (MIP-3a), CCL21 (MTP-2), SLC, exodus-2, CCL22 (MDC/STC-I), CCL23 (MPIF-1), CCL24 (MPIF-2/eotaxin-2), CCL25 (TECK), CCL26 (eotaxin-3), CCL27 (CTACK/ILC), CCL28, CCL3 (MTP-la), CCL4 (MDP-lb), CCL5 (RANTES), CCL7 (MCP-3), CCL8 (mcp-2), CCNAI, CCNA2, CCNDI, CCNEI, CCNE2, CCRI (CKRI/HM145), CCR2 (mcp-IRB/RA), CCR3 (CKR3/CMKBR3), CCR4, CCR5 (CMKBR5/ChemR13), CCR6 (CMKBR6/CKR-L3/STRL22/DRY6), CCR7 (CKR7/EBII), CCR8 (CMKBR8/TERI/CKR-LI), CCR9 (GPR-9-6), CCRLI (VSHKI), CCRL2 (L-CCR), CD164, CD19, CD105, CDIC, CD20, CD200, CD22, CD24, CD28, CD3, CD33, CD37, CD38, CD3E, CD3G, CD3Z, CD4, CD40, CD40L, CD44, CD47, CD45RB, CD52, CD56, CD69, CD70, CD72, CD73, CD74, CD79A, CD79B, CD8, CD80, CD81, CD83, CD86, CDHI (E-cadherin), CDH10, CDH12, CDH13, CDH18, CDH19, CDH20, CDH5, CDH7, CDH8, CDH9, CDK2, CDK3, CDK4, CDK5, CDK6, CDK7, CDK9, CDKNIA (p21Wapl/Cipl), CDKNIB (p27Kipl), CDKNIC, CDKN2A(P16INK4a), CDKN2B, CDKN2C, CDKN3, CEA, CEBPB, CERI, CHGA, CHGB, chitinase, CHST10, CKLFSF2, CKLFSF3, CKLFSF4, CKLFSF5, CKLFSF6, CKLFSF7, CKLFSF8, CLDN3, CLDN7, Claudin18.2, CLN3, CLU (clusterin), c-Met, CMKLRI, CMKORI (RDCI), CNRI, COL18A1, COLIAI, COL4A3, COL6A1, CR2, Cripto, CRP, CSFI(M-CSF), CSF2 (GM-CSF), CSF3 (GCSF), CTGF, CTLA4, CTNNBI (b-catenin), CTSB (cathepsin B), CX3CL1 (SCYDI), CX3CR1 (V28), CXCLI (GROI), CXCL10 (IP-10), CXCLII (l-TAC/IP-9), CXCL12 (SDFI), CXCL13, CXCL14, CXCL16, CXCL2, (GR02), CXCL3 (GR03), CXCL5 (ENA-78/LIX), CXCL6 (GCP-2), CXCL9 (MIG), CXCR3 (GPR9/CKR-L2), CXCR4, CXCR6 (TYMSTR/STRL33Bonzo), CYB5, CYCI, CYSLTRI, DAB2IP, DES, DKFZp451J0118, DNCLI, DPP4, E2F1, ECGFI, EDGI, EFNAI, EFNA3, EFNB2, EGF, EGFR, ELAC2, ENG, EN01, EN02, EN03, EpCAM, EPHB4, EPO, ERBB2 (Her-2), ERBB3 (Her-3), ERBB4 (Her-4), EREG, ERK8, ESRI, ESR2, F3 (TF), FADD, FasL, FASN, FCERIA, FCER2, FCGR3A, FGF, FGFI (aFGF), FGF10, FGF11, FGF12, FGF12B, FGF13, FGF14, FGF16, FGF17, FGF18, FGF19, FGF2(bFGF), FGF20, FGF21, FGF22, FGF23, FGF3 (int-2), FGF4 (HST), FGF5, FGF6 (HST-2), FGF7 (KGF), FGF8, FGF9, FGFR3, FIGF (VEGFD), FELI (EPSILON), FILI (ZETA), FLJ12584, FLJ25530, FLRTI (fibronectin), FLTI, FOS, FOSLI (FRA-I), FY (DARC), G250, GABRP (GABAa), GAGEBI, GAGECI, GALNAC4S-6ST, GAT A3, GDF5, GFI 1, GGT1, GM-CSF, GNASI, GNRHI, GPR2 (CCRIO), GPR31, GPR44, GPR81 (FKSG80), GRCCIO (CIO), GRP, GPNMB, GSN (villin), GSTPI, HAVCR2, HDAC4, HDAC5, HDAC7A, HDAC9, HGF, HIFIA, HDPI, histamine and histamine receptors, HLA-A, HLA-DRA, HM74, HMOXI, HUMCYT2A, ICEBERG, ICOSL, ID2, IFN-a, IFNAI, IFNA2, IFNA4, IFNA5, IFNA6, IFNA7, IFNB1, IFNγ, DFNWI, IGBPI, IGFI, IGFIR, IGF2, IGFBP2, IGFBP3, IGFBP6, IL-I, IL10, MORA, IL10RB, II,11, IL11RA, IL-12, IL12A, IL12B, IL12RB1, IL12RB2, IL13, IL13RA1, IL13RA2, IL14, IL15, IL15RA, IL16, IL17, IL17B, IL17C, IL17R, IL18, IL18BP, IL18R1, IL18RAP, IL19, IL1A, IL1B, ILIF10, IL1F5, IL1F6, IL1F7, IL1F8, IL1F9, II,1HYI, IL1R1, IL1R2, IL1RAP, IL1RAPL1, IL1RAPL2, IL1RL1, IL1RL2, ILIRN, IL2, IL20, IL20RA, IL21R, IL22, IL22R, IL22RA2, IL23, IL24, IL25, IL26, IL27, I L28A, IL28B, IL29, IL2RA, IL2RB, IL2RG, IL3, IL30, IL3RA, IL4, IL4R, IL5, IL5RA, IL6, IL6R, IL6ST (glycoprotein 130), EL7, EL7R, EL8, EphA2, IL8RA, DL8RB, IL8RB, DL9, DL9R, DLK, INHA, INHBA, INSL3, INSL4, IRAKI, Integrin, ERAK2, ITGAI, ITGA2, ITGA3, ITGA6 (a6 integrin), ITGAV, ITGB3, ITGB4 (b4 integrin), JAGI, JAKI, JAK3, JUN, K6HF, KAN, KDR, KITLG, KLF5 (GC Box BP), KLF6, KLKIO, KLK12, KLK13, KLK14, KLK15, KLK3, KLK4, KLK5, KLK6, KLK9, KRT1, KRT19 (keratin19), KRT2A, KHTHB6 (hair-specific type H keratin), LAG3, LAMAS, LEP (leptin), Lewis Y, Lingo-p75, Lingo-Troy, LPS, LTA (TNF-b), LTB, LTB4R (GPR16), LTB4R2, LTBR, MACMARCKS, MAG or Omgp, MAP2K7 (c-Jun), MDK, Mesothelin, MIBI, MUCl, midkine, MEF, MIP-2, MKI67, (Ki-67), MMP2, MMP9, MS4A1, MSMB, MT3 (metallothionectin-lll), MTSSI, MUCI (mucin), MYC, MYD88, NCK2, neurocan, NFKBI, NFKB2, NGFB (NGF), NGFR, NgR-Lingo, NgR-Nogo66 (Nogo), NgR-p75, NgR-Troy, NMEI (NM23A), N0X5, NPPB, NROBI, NR0B2, NRIDI, NR1D2, NR1H2, NR1H3, NR1H4, NR1I2, NR1I3, NR2C1, NR2C2, NR2E1, NR2E3, NR2F1, NR2F2, NR2F6, NR3C1, NR3C2, NR4A1, NR4A2, NR4A3, NR5A1, NR5A2, NR6A1, NRPI, NRP2, NT5E, NTN4, ODZI, OPRDI, P2RX7, PAP, PARTI, PATE, PAWR, PCA3, PCNA, PCSK9, PD-1, PD-L1, PDGFA, PDGFB, PECAMI, PF4(CXCL4), PGF, PGR, phosphacan, PIAS2, PIK3CG, PLAU (uPA), PLG, PLXDCI, PPBP (CXCL7), PPID, PRI, PRKCQ, PRKDI, PRL, PROC, PROK2, PSAP, PSCA, PSMA, PTAFR, PTEN, PTGS2 (COX-2), PTN, RAC2 (p21Rac2), RARB, RGSI, RGS13, RGS3, RNFIIO (ZNF144), ROB02, S100A2, SCGB1D2 (lipophilinB), SCGB2A1 (mammaglobin 2), SCGB2A2 (mamnnaglobin 1), SCYEI (endothelial monocyte-activating cytokine), SDF2, SERPINAI, SERPINA3, SERP1NB5 (maspin), SERPINEI (PAI-I), SERPDMF1, SHBG, SLA2, SLC2A2, SLC33A1, SLC43A1, SLC44A4, SLIT2, SOST, SPPI, SPRRIB (Sprl), ST6GAL1, STABI, STAT6, STEAP, STEAP2, TB4R2, TBX21, TCPIO, TDGFI, TEK, TGFA, TGFBI, TGFBIII, TGFB2, TGFB3, TGFBI, TGFBRI, TGFBR2, TGFBR3, THIL, THBSI (thrombospondin-1), THBS2, THBS4, THPO, TIE (Tie-1), TIGHT, Tenascin-C, TMP3, tissue factor, TLRIO, TLR2, TLR3, TLR4, TLR5, TLR6, TLR7, TLR8, TLR9, TNF, TNF-a, TNFAEP2 (B94), TNFAIP3, TNFRSFIIA, TNFRSFIA, TNFRSFIB, TNFRSF21, TNFRSF5, TNFRSF6 (Fas), TNFRSF7, TNFRSF8, TNFRSF9, TNFSFIO (TRAIL), TNFSFI 1 (TRANCE), TNFSF12 (AP03L), TNFSF13 (April), TNFSF13B, TNFSF14 (HVEM-L), TNFSF15 (VEGI), TNFSF18, TNFSF4 (OX40 ligand), TNFSF5 (CD40 ligand), TNFSF6 (FasL), TNFSF7 (CD27 ligand), TNFSF8 (CD30 ligand), TNFSF9 (4-1BB ligand), TOLLIP, Toll-like receptors, TOP2A (topoisomerase Ea), TP53, TPMI, TPM2, TRADD, TRAFI, TRAF2, TRAF3, TRAF4, TRAF5, TRAF6, TREMI, TREM2, TRPC6, TSLP, TWEAK, VEGF, VEGFB, VEGFC, VEGFR, versican, VHL C5, VLA-4, XCLI (lymphotactin), XCL2 (SCM-lb), XCRI (GPR5/CCXCRI), YYI, ZFPM2 and thrombin.

Illustratively, the antibody is selected from the group consisting of trastuzumab, pertuzumab, nimotuzumab, enoblituzumab, emibetuzumab, inotuzumab, pinatuzumab, brentuximab, gemtuzumab, bivatuzumab, lorvotuzumab, cBR96, glematumamab and an anti-Claudin18.2 antibody, wherein the heavy chain of the anti-Claudin18.2 antibody is set forth in SEQ ID NO: 49, and the light chain is set forth in SEQ ID NO: 47.

"Conservative modification" or "conservative replacement or substitution" refers to replacement of amino acids in a protein with other amino acids having similar characteristics (e.g., charge, side-chain size, hydrophobicity/hydrophilicity, or backbone conformation and rigidity), so that changes can be frequently made without changing the biological activity of the protein. Those skilled in the art know that, generally speaking, a single amino acid replacement in a non-essential region of a polypeptide does not substantially change the biological activity (see, e.g., Watson et al. (1987) Molecular Biology of the Gene, The Benjamin/Cummings Pub. Co., p224, (4th edition)). In addition, the replacement of amino acids with similar structure or function is unlikely to disrupt the biological activity. Exemplary conservative substitutions are set forth below.

**Table 1. Amino acid conservative substitutions**

| Original residue | Conservative substitution |
|---|---|
| Ala (A) | Gly; Ser |
| Arg (R) | Lys; His |
| Asn (N) | Gln; His; Asp |
| Asp (D) | Glu; Asn |
| Cys (C) | Ser; Ala; Val |
| Gln (Q) | Asn; Glu |
| Glu (E) | Asp; Gln |
| Gly (G) | Ala |
| His (H) | Asn; Gln |
| Ile (I) | Leu; Val |
| Leu (L) | Ile; Val |
| Lys (K) | Arg; His |
| Met (M) | Leu; Ile; Tyr |
| Phe (F) | Tyr; Met; Leu |
| Pro (P) | Ala |
| Ser (S) | Thr |
| Thr (T) | Ser |
| Trp (W) | Tyr; Phe |
| Tyr (Y) | Trp; Phe |
| Val (V) | Ile; Leu |

The term "terminal extension ratio" described herein refers to the ratio of expression products having a terminal extension sequence among an expression product population. Expression products having a terminal extension sequence are variants of the expression product of interest; they comprise the sequence of interest and other amino acid residues attached thereto as well. Illustratively, in an antibody light chain expression product, the terminal extension ratio of the expression product is 1% if 1% of the light chain sequence variants have other amino acid residues attached at the N-terminus. When the expression product population contains an expression product having a terminal extension sequence, it means that the expression product population has terminal heterogeneity. Illustratively, the terminal extension sequences in the present disclosure are derived from the residue of signal peptide amino acids.

The composition of the present disclosure comprises a desired expression product of interest and an expression product having a terminal extension sequence. The presence of amino-terminal extension in the composition can be detected by a variety of analytical techniques, including but not limited to, N-terminal sequence analysis, assays for charge heterogeneity (e.g., cation exchange chromatography or capillary zone electrophoresis), mass spectrometry, peptide mapping assay, and the like. The amount of the antibody variant in the composition generally ranges from an amount that constitutes the lower detection limit of any assay (e.g., cation exchange analysis) used to detect the variant to an amount less than the amount of the main species antibody. About 3% or less (e.g., about 3%, 2.5%, 2%, 1.5%, 1%, 0.5% or 0%) of the antibody light chains or antibody heavy chains in the composition comprise an amino-terminal extension. Such percentages can be determined by mass spectrometry or peptide mapping assay. When the proportion of the heterologous polypeptides containing the terminal extension exceeds 1% of the total amount of the sample, the heterologous polypeptides can be effectively identified based on peptide mapping assay, and simultaneously, the corresponding peak in the mass spectrogram for molecular weight after reduction is quantitatively identified.

The details of one or more embodiments of the present disclosure are set forth in the specification above. Although any methods and materials similar or identical to those described herein can be used in the practice or testing of the present disclosure, the methods and materials are described below. Other features, objects and advantages of the present disclosure will be apparent from the specification and the claims. In the specification and claims, singular forms include plural referents unless otherwise indicated clearly in the context. Unless otherwise defined, all technical and scientific terms used herein have the meanings generally understood by those of ordinary skill in the art to which the present disclosure belongs. All the patents and publications cited in the specification are incorporated by reference. The following examples are set forth to more fully illustrate the optional embodiments of the present disclosure. These examples should not be construed in any way as limiting the scope of the present disclosure, which is defined by the claims.

### Examples

The present disclosure is further described with reference to the following examples, which, however, do not limit the scope of the present disclosure. The experimental methods in the examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturers of the starting materials or commercial products. Reagents without specific origins indicated are commercially available conventional reagents.

### Example 1: Preparation of Anti-Claudin18.2 Antibody

### Example 1-1: Construction of Cell Strain Highly Expressing Claudinl8.2

pCDH-hClaudin18.2 lentiviral expression vector plasmids, pVSV-G and pCMV-dR8.91 lentiviral system packaging vectors were transfected into viral packaging cells 293T using Lipofectamine 3000 transfection reagent. The medium supernatant containing viruses was collected, filtered and centrifuged at ultra-high speed. The human gastric signet ring cell carcinoma cell strain NUGC4 was infected with the concentrated virus, screened using puromycin for two to three weeks and then subjected to FACS single-cell sorting.

Claudin18.2 expression levels were determined according to tumor IHC scores. Cells with a Claudin18.2 expression level similar to that of a tumor with a tumor IHC score of 3 points were considered cells with high expression, and cells with a Claudin18.2 expression level similar to that of a tumor with a tumor IHC score of 2 points were considered cells with moderate expression. According to the Claudin18.2 expression levels on the surface of NUGC4 cells infected by lentivirus determined by FACS, NUGC4/hClaudin18.2 monoclonal cell strains with high Claudin18.2 expression were selected. At the same time, the Claudin18.2 expression level on the surface of wild-type NUGC4 cells were also determined by FACS, and NUGC4 clonal cell strains with moderate Claudin18.2 expression were selected. The wild-type NUGC4 cells were cells with low Claudin18.2 expression.

The selected monoclonal cell strains were expanded and preserved by freezing for subsequent experiments.
Sequence of Claudin18.2, Genbank NP_001002026: (SEQ ID NO: 1) 
MAVTACQGLGFVVSLIGIAGIIAATCMDQWSTQDLYNNPVTAVFNYQGLWRSCV
RESSGFTECRGYFTLLGLPAMLQAVRALMIVGIVLGAIGLLVSIFALKCIRIGSME
DSAKANMTLTSGIMFIVSGLCAIAGVSVFANMLVTNFWMSTANMYTGMGGMV
QTVQTRYTFGAALFVGWVAGGLTLIGGVMMCIACRGLAPEETNYKAVSYHASG
H S V A Y K P G G F K A S T G F G S N T K N K K I Y D G G A R T E D E V Q S Y P S K H D Y V.
DNA sequence of Claudin18.2: (SEQ ID NO: 2)

1 AGAATTGCGC TGTCCACTTG TCGTGTGGCT CTGTGTCGAC ACTGTGCGCC ACCATGGCCG
61 TGACTGCCTG TCAGGGCTTG GGGTTCGTGG TTTCACTGAT TGGGATTGCG GGCATCATTG
121 CTGCCACCTG CATGGACCAG TGGAGCACCC AAGACTTGTA CAACAACCCC GTAACAGCTG
181 TTTTCAACTA CCAGGGGCTG TGGCGCTCCT GTGTCCGAGA GAGCTCTGGC TTCACCGAGT

241 GCCGGGGCTA CTTCACCCTG CTGGGGCTGC CAGCCATGCT GCAGGCAGTG CGAGCCCTGA
301 TGATCGTAGG CATCGTCCTG GGTGCCATTG GCCTCCTGGT ATCCATCTTT GCCCTGAAAT
361 GCATCCGCAT TGGCAGCATG GAGGACTCTG CCAAAGCCAA CATGACACTG ACCTCCGGGA
421 TCATGTTCAT TGTCTCAGGT CTTTGTGCAA TTGCTGGAGT GTCTGTGTTT GCCAACATGC
481 TGGTGACTAA CTTCTGGATG TCCACAGCTA ACATGTACAC CGGCATGGGT GGGATGGTGC
541 AGACTGTTCA GACCAGGTAC ACATTTGGTG CGGCTCTGTT CGTGGGCTGG GTCGCTGGAG
601 GCCTCACACT AATTGGGGGT GTGATGATGT GCATCGCCTG CCGGGGCCTG GCACCAGAAG
661 AAACCAACTA CAAAGCCGTT TCTTATCATG CCTCAGGCCA CAGTGTTGCC TACAAGCCTG
721 GAGGCTTCAA GGCCAGCACT GGCTTTGGGT CCAACACCAA AAACAAGAAG ATATACGATG
781 GAGGTGCCCG CACAGAGGAC GAGGTACAAT CTTATCCTTC CAAGCACGAC TATGTGTAAT
841 GCTCTAAGAC CTCTCAGCAC GGGCGGAAGA AACTCCCGGA GAGCTCACCC AAAAAACAAG
901 GAGATCCCAT CTAGATTTCT TCTTGCTTTT GACTCACAGC TGGAAGTTAG AAAAGCCTCG
961 ATTTCATCTT TGGAGAGGCC AAATGGTCTT AGCCTCAGTC TCTGTCTCTA AATATTCCAC
1021 CATAAAACAG CTGAGTTATT TATGAATTAG AGGCTATAGC TCACATTTTC AATCCTCTAT
1081 TTCTTTTTTT AAATATAACT TTCTACTCTG ATGAGAGAAT GTGGTTTTAA TCTCTCTCTC
1141 ACATTTTGAT GATTTAGACA GACTCCCCCT CTTCCTCCTA GTCAATAAAC CCATTGATGA
1201 TCTATTTCCC AGCTTATCCC CAAGAAAACT TTTGAAAGGA AAGAGTAGAC CCAAAGATGT
1261 TATTTTCTGC TGTTTGAATT TTGTCTCCCC ACCCCCAACT TGGCTAGTAA TAAACACTTA
1321 CTGAAGAAGA AGCAATAAGA GAAAGATATT TGTAATCTCT CCAGCCCATG ATCTCGGTTT
1381 TCTTACACTG TGATCTTAAA AGTTACCAAA CCAAAGTCAT TTTCAGTTTG AGGCAACCAA
1441 ACCTTTCTAC TGCTGTTGAC ATCTTCTTAT TACAGCAACA CCATTCTAGG AGTTTCCTGA
1501 GCTCTCCACT GGAGTCCTCT TTCTGTCGCG GGTCAGAAAT TGTCCCTAGA TGAATGAGAA
1561 AATTATTTTT TTTAATTTAA GTCCTAAATA TAGTTAAAAT AAATAATGTT TTAGTAAAAT
1621 GATACACTAT CTCTGTGAAA TAGCCTCACC CCTACATGTG GATAGAAGGA AATGAAAAAA
1681 TAATTGCTTT GACATTGTCT ATATGGTACT TTGTAAAGTC ATGCTTAAGT ACAAATTCCA
1741 TGAAAAGCTC ACTGATCCTA ATTCTTTCCC TTTGAGGTCT CTATGGCTCT GATTGTACAT
1801 GATAGTAAGT GTAAGCCATG TAAAAAGTAA ATAATGTCTG GGCACAGTGG CTCACGCCTG
1861 TAATCCTAGC ACTTTGGGAG GCTGAGGAGG AAGGATCACT TGAGCCCAGA AGTTCGAGAC
1921 TAGCCTGGGC AACATGGAGA AGCCCTGTCT CTACAAAATA CAGAGAGAAA AAATCAGCCA
1981 GTCATGGTGG CCTACACCTG TAGTCCCAGC ATTCCGGGAG GCTGAGGTGG GAGGATCACT
2041 TGAGCCCAGG GAGGTTGGGG CTGCAGTGAG CCATGATCAC ACCACTGCAC TCCAGCCAGG
2101 TGACATAGCG AGATCCTGTC TAAAAAAATA AAAAATAAAT AATGGAACAC AGCAAGTCCT
2161 AGGAAGTAGG TTAAAACTAA TTCTTTAAAA AAAAAAAAAA GTTGAGCCTG AATTAAATGT
2221 AATGTTTCCA AGTGACAGGT ATCCACATTT GCATGGTTAC AAGCCACTGC CAGTTAGCAG
2281 TAGCACTTTC CTGGCACTGT GGTCGGTTTT GTTTTGTTTT GCTTTGTTTA GAGACGGGGT
2341 CTCACTTTCC AGGCTGGCCT CAAACTCCTG CACTCAAGCA ATTCTTCTAC CCTGGCCTCC
2401 CAAGTAGCTG GAATTACAGG TGTGCGCCAT CACAACTAGC TGGTGGTCAG TTTTGTTACT
2461 CTGAGAGCTG TTCACTTCTC TGAATTCACC TAGAGTGGTT GGACCATCAG ATGTTTGGGC
2521 AAAACTGAAA GCTCTTTGCA ACCACACACC TTCCCTGAGC TTACATCACT GCCCTTTTGA
2581 GCAGAAAGTC TAAATTCCTT CCAAGACAGT AGAATTCCAT CCCAGTACCA AAGCCAGATA
2641 GGCCCCCTAG GAAACTGAGG TAAGAGCAGT CTCTAAAAAC TACCCACAGC AGCATTGGTG
2701 CAGGGGAACT TGGCCATTAG GTTATTATTT GAGAGGAAAG TCCTCACATC AATAGTACAT
2761 ATGAAAGTGA CCTCCAAGGG GATTGGTGAA TACTCATAAG GATCTTCAGG CTGAACAGAC
2821 TATGTCTGGG GAAAGAACGG ATTATGCCCC ATTAAATAAC AAGTTGTGTT CAAGAGTCAG
2881 AGCAGTGAGC TCAGAGGCCC TTCTCACTGA GACAGCAACA TTTAAACCAA ACCAGAGGAA
2941 GTATTTGTGG AACTCACTGC CTCAGTTTGG GTAAAGGATG AGCAGACAAG TCAACTAAAG
3001 AAAAAAGAAA AGCAAGGAGG AGGGTTGAGC AATCTAGAGC ATGGAGTTTG TTAAGTGCTC
3061 TCTGGATTTG AGTTGAAGAG CATCCATTTG AGTTGAAGGC CACAGGGCAC AATGAGCTCT
3121 CCCTTCTACC ACCAGAAAGT CCCTGGTCAG GTCTCAGGTA GTGCGGTGTG GCTCAGCTGG
3181 GTTTTTAATT AGCGCATTCT CTATCCAACA TTTAATTGTT TGAAAGCCTC CATATAGTTA
3241 GATTGTGCTT TGTAATTTTG TTGTTGTTGC TCTATCTTAT TGTATATGCA TTGAGTATTA
3301 ACCTGAATGT TTTGTTACTT AAATATTAAA AACACTGTTA TCCTACAGTT

### Example 1-2: Production of Anti-Human Claudinl8.2 Monoclonal Antibody

### 1. Immunization

The anti-human Claudin18.2 monoclonal antibody was produced by immunizing mice. Laboratory SJL white mice, female, 6-8 weeks of age (Beijing Vital River Laboratory Animal Technology Co., Ltd., animal production license number: SCXK(Beijing)2012-0001). Feeding environment: SPF grade. The purchased mice are fed in a laboratory environment for 1 week, in a 12/12 hour light/dark cycle, at a temperature of 20-25 °C, with humidity at 40-60%. The acclimatized mice were immunized according to the following scheme. The antigens for immunization were huClaudin18.2-HEK293 cells (a HEK-293 cell strain stably transfected with human Claudin18.2 plasmid).

Immunization scheme: Prior to the first cell immunization, each mouse was intraperitoneally (IP) injected with 0.1 mL of TiterMax^{®} Gold Adjuvant (Sigma Cat No. T2684), and, a half hour later, with 0.1 mL of normal saline-diluted cellular fluid at a concentration of 1×10⁸/mL. The cells were uniformly pipetted, and then inoculation was performed at days 0, 14, 28, 42 and 56. Blood was collected at days 21, 35, 49 and 63, and the antibody titer in mouse serum was determined by ELISA. After 4-5 immunizations, mice in which the antibody titer in serum was high and was reaching a plateau were selected for splenocyte fusion. The mice were immunized with a booster dose of 1×10⁷ cells by intraperitoneal injection (IP) 3 days prior to splenocyte fusion.

### 2. Splenocyte fusion

Spleen lymphocytes and myeloma cells, Sp2/0 cells (ATCC^{®} CRL-8287^{™}), were fused by following a PEG-mediated fusion procedure to obtain hybridoma cells. The hybridoma cells were resuspended in complete medium (IMDM medium containing 20% FBS, 1× HAT and 1× OPI) at a density of 0.5-1×10⁶/mL and seeded in a 96-well plate at 100 µL/well. The plate was incubated at 37 °C with 5% CO₂ for 3-4 days, supplemented with HAT complete medium at 100 µL/well, and incubated for another 3-4 days to form clones. The supernatant was removed and HT complete medium (IMDM medium containing 20% FBS, 1× HT and 1×OPI) was added at 200 µL/well. The plate was incubated at 37 °C with 5% CO₂ for 3 days, followed by an ELISA assay.

### 3. Screening of hybridoma cells

The culture supernatants were assayed using a combined ELISA method according to the density the hybridoma cells were growing at. Cells that had good binding capacity to huClaudin18.2-HEK293 cells but were not bound to HEK293 were selected, expanded and frozen in time. Subcloning was performed two to three times to obtain single-cell clones.

A cell binding assay was performed for each cell subcloning. Hybridoma clones were obtained by the above screening process, and antibodies were further prepared using a serum-free cell culture method. The antibodies were purified for use in the test examples.

### Example 1-3: Humanization of Murine Antibodies

Monoclonal hybridoma cell strains mAb1901 and mAb1902 with high *in vitro* activity were selected. The monoclonal antibody sequences therein were cloned, followed by humanization, recombinant expression and activity evaluation.

The cloning of a sequence from hybridoma is as follows. Hybridoma cells at logarithmic growth phase were harvested, and the RNA was extracted using Trizol (Invitrogen, 15596-018) (following the procedures in the kit instructions) and reverse transcribed (PrimeScript^{™} Reverse Transcriptase, Takara, cat # 2680A). The cDNA obtained by reverse transcription was amplified by PCR using mouse Ig-Primer Set (Novagen, TB326 Rev.B 0503) and then sent for sequencing by a sequencing company. The amino acid sequences corresponding to the obtained DNA sequences are set forth in SEQ ID NOs: 3-6:
Murine heavy chain variable region of mAb1901 (SEQ ID NO: 3) 
EVQLMESGGGLVKPGGSLKLSCAASGFTFSDYGIHWVRQAPEMGLEWIAYISR
GSSTIYYADTVKGRFTMSRDNAKNTLFLQMTSLRSEDTAMYYCARGGYDTRN
AMDYWGQGTSVTVSS.
Murine light chain variable region of mAb1901 (SEQ ID NO: 4) 
DIVMTQSPSSLSVSAGEKVTMSCKSSQSLLNSGNQKNYLAWYQQKPGQPPKLLI
YGASTRASGVPDRFTGSGSGTDFTLTISSVQAEDLAIYHCQNDLYYPLTFGAGTK
LELK.
Murine heavy chain variable region of mAb1902 (SEQ ID NO: 5) 
EVQLQESGAELVKPGASVKLSCKASGYIFTSYWMHWVKQRPGQGLEWIGMIHP
NSGSTNYNEKFKGKATLTLDKSSSTAYMQLSSLPSEDSAVYYCARLKTGNSFDY
WGQGTTLTVSS.
Murine light chain variable region ofmAb1902 (SEQ ID NO: 6)

DIVLTQSPSSLTVTAGEKVTMSCKSSQSLLNSGNQKNYLTWYQQKPGQPPKLLI
YWASTRESGVPDRFTGSGSGTDFTLTISSVQAEDLAIYYCQNAYTYPFTFGSGTK
LEIK.

The above murine heavy chain and light chain variable regions were linked to the heavy chain constant region of human IgG1 antibody and the human κ light chain constant region described below, respectively, to form chimeric antibodies ch1901 and ch1902. The constant regions were selected from the group consisting of the following sequences:
Heavy chain constant region of human IgG1 antibody: (SEQ ID NO: 7) 






.
Human κ light chain constant region: (SEQ ID NO: 8)


.

Murine monoclonal antibodies are humanized according to methods disclosed in many publications in the art. Briefly, human constant domains were used in place of parent (murine antibody) constant domains, and human germline antibody sequences were selected, based on the homology of the murine and human antibodies, for CDR grafting. The present disclosure selects candidate molecules with good activity for humanization, and the results are as follows.

### 1. CDRs of murine antibodies

The amino acid residues of the VH/VL CDRs in Table 2 were identified using the Kabat numbering scheme and annotated.

The CDR sequences of the murine antibodies are described in Table 2:

**Table 2. CDR sequences of murine antibodies**

| **Antibody** | mAb 1901 |
|---|---|
| **HCDR1** | DYGIH (SEQ ID NO: 9) |
| **HCDR2** | YISRGSSTIYYADTVKG (SEQ ID NO: 10) |
| **HCDR3** | GGYDTRNAMDY (SEQ ID NO: 11) |
| **LCDR1** | KSSQSLLNSGNQKNYLA (SEQ ID NO: 12) |
| **LCDR2** | GASTRAS (SEQ ID NO: 13) |
| **LCDR3** | QNDLYYPLT (SEQ ID NO: 14) |

| **Antibody** | mAb 1902 |
|---|---|
| **HCDR1** | SYWMH (SEQ ID NO: 15 ) |
| **HCDR2** | MIHPNSGSTNYNEKFKGR (SEQ ID NO: 16) |
| **HCDR3** | LKTGNSFDY (SEQ ID NO: 17) |
| **LCDR1** | KSSQSLLNSGNQKNYLT (SEQ ID NO: 18) |
| **LCDR2** | WASTRES (SEQ ID NO: 19) |
| **LCDR3** | QNAYTYPFT (SEQ ID NO: 20 ) |

### 2. Selection of human germline FR region sequences

Based on the typical structure of the murine antibody VH/VL CDR obtained, the heavy chain and light chain variable region sequences were compared with an antibody Germline database to obtain a human germline template with high homology. The human germline light chain framework region was derived from a human κ light chain gene.

### 2.1. Humanization of mAb1901 and reverse mutation design

A suitable human antibody germline was selected for humanization of mAb1901 murine antibody. The CDRs of murine antibody mAb1901 were grafted into the selected humanization template to obtain a humanized variable region (the sequence of the humanized heavy chain variable region was SEQ ID NO: 24, and the sequence of the light chain variable region was SEQ ID NO: 21), followed by recombination with an IgG constant region to form a complete antibody. Meanwhile, reverse mutations were introduced into the FR region in the V region of the humanized antibody. Exemplary reverse mutations and combinations thereof are as follows:

**Table 3. Humanized antibody of mAb1901 and reverse mutations***

| Light chain variable regions of humanized antibody of mAb1901 | | Heavy chain variable regions of humanized antibody of mAb1901 | |
|---|---|---|---|
| VL1 | None | VH1 | None |
| VL2 | N22S | VH2 | N82T |
| VL3 | N22S, V85I, Y87H | VH3 | V48I, N82T |
| | | VH4 | I69M, N82T |

| | | | |
|---|---|---|---|
| * All amino acid positions in the table are numbered according to the Kabat numbering scheme; in N82T of the heavy chain variable region, 82 refers to position 82A according to the Kabat scheme. | | | |

**Table 4. Light chain and heavy chain variable region sequences of humanized antibody of mAb 1901**

| Name of variable region (SEQ ID NO:) | Sequence |
|---|---|
| VL1 (SEQ ID NO: 21) | DIVMTQSPDSLAVSLGERATINCKSSQSLLNSGNQKNYLAWY QQKPGQPPKLLIYGASTRASGVPDRFSGSGSGTDFTLTISSLQ AEDVAVYYCQNDLYYPLTFGQGTKLEIK |
| VL2 (SEQ ID NO: 22) | DIVMTQSPDSLAVSLGERATISCKSSQSLLNSGNQKNYLAWY QQKPGQPPKLLIYGASTRASGVPDRFSGSGSGTDFTLTISSLQ AEDVAVYYCQNDLYYPLTFGQGTKLEIK |
| VL3 (SEQ ID NO: 23) | DIVMTQSPDSLAVSLGERATISCKSSQSLLNSGNQKNYLAWY QQKPGQPPKLLIYGASTRASGVPDRFSGSGSGTDFTLTISSLQ AEDVAIYHCQNDLYYPLTFGQGTKLEIK |
| VH1 (SEQ ID NO: 24) | EVQLVESGGGLVQPGGSLRLSCAASGFTFSDYGIHWVRQAP GKGLEWVAYISRGSSTIYYADTVKGRFTISRDNAKNSLYLQM NSLRAEDTAVYYCARGGYDTRNAMDYWGQGTTVTVSS |
| VH2 (SEQ ID NO: 25) | EVQLVESGGGLVQPGGSLRLSCAASGFTFSDYGIHWVRQAP GKGLEWVAYISRGSSTIYYADTVKGRFTISRDNAKNSLYLQM TSLRAEDTAVYYCARGGYDTRNAMDYWGQGTTVTVSS |
| VH3 (SEQ ID NO: 26) | EVQLVESGGGLVQPGGSLRLSCAASGFTFSDYGIHWVRQAP GKGLEWIAYISRGSSTIYYADTVKGRFTISRDNAKNSLYLQM TSLRAEDTAVYYCARGGYDTRNAMDYWGQGTTVTVSS |
| VH4 (SEQ ID NO: 27) | EVQLVESGGGLVQPGGSLRLSCAASGFTFSDYGIHWVRQAP GKGLEWVAYISRGSSTIYYADTVKGRFTMSRDNAKNSLYLQ MTSLRAEDTAVYYCARGGYDTRNAMDYWGQGTTVTVSS |

The corresponding heavy chain variable region in the table above could be linked to the human IgG1 heavy chain constant region set forth in SEQ ID NO: 7 to form a heavy chain of a full-length antibody, and the light chain variable region was linked to the human κ light chain constant region set forth in SEQ ID NO: 8 to form a light chain of a full-length antibody. In other embodiments, the heavy chain variable region and the light chain variable region may also be linked to other heavy chain constant regions and light chain constant regions, respectively, to form a full-length antibody.

### 2.2. Humanization of mAb1902 and reverse mutation design

A suitable human antibody germline was selected for humanization of mAb1902 murine antibody. The CDRs of murine antibody mAb1902 were grafted into the selected humanization template to obtain a humanized variable region (the sequence of the humanized heavy chain variable region was SEQ ID NO: 31, and the sequence of the light chain variable region was SEQ ID NO: 28), followed by recombination with an IgG constant region to form a complete antibody. Meanwhile, reverse mutations were introduced into the FR region in the V region of the humanized antibody. Exemplary reverse mutations and combinations thereof are as follows:

**Table 5. Humanized antibody of mAb1902 and reverse mutation design therefor***

| Light chain variable regions of humanized antibody of mAb 1902 | | Heavy chain variable regions of humanized antibody of mAb 1902 | |
|---|---|---|---|
| VL11 | None | VH11 | None |
| VL12 | M4L | VH12 | I69L, R71L, T73K |
| VL13 | M4L, N22S | VH13 | M48I, R66K, V67A, I69L, R71L, T73K |
| | | VH14 | R38K, A40R, M48I, R66K, V67A, I69L, R71L, T73K |

| | | | |
|---|---|---|---|
| * All amino acid positions in the table are numbered according to the Kabat numbering scheme. | | | |

**Table 6. Light chain and heavy chain variable region sequences of humanized antibody of mAb 1902**

| Name of variable region (SEQ ID NO:) | Sequence |
|---|---|
| VL11 (SEQ ID NO: 28) | DIVMTQSPDSLAVSLGERATINCKSSQSLLNSGNQKNYLTWYQQ KPGQPPKLLIYWASTRESGVPDRFSGSGSGTDFTLTISSLQAEDVA VYYCQNAYTYPFTFGQGTKLEIK |
| VL12 (SEQ ID NO: 29) | DIVLTQSPDSLAVSLGERATINCKSSQSLLNSGNQKNYLTWYQQK PGQPPKLLIYWASTRESGVPDRFSGSGSGTDFTLTISSLQAEDVAV YYCQNAYTYPFTFGQGTKLEIK |
| VL13 (SEQ ID NO: 30) | DIVLTQSPDSLAVSLGERATISCKSSQSLLNSGNQKNYLTWYQQK PGQPPKLLIYWASTRESGVPDRFSGSGSGTDFTLTISSLQAEDVAV YYCQNAYTYPFTFGQGTKLEIK |
| VH11 (SEQ ID NO: 31) | EVQLVQSGAEVKKPGASVKVSCKASGYTFTSYWMHWVRQAPG QRLEWMGMIHPNSGSTNYNEKFKGRVTITRDTSASTAYMELSSL RSEDTAVYYCARLKTGNSFDYWGQGTTVTVSS |
| VH12 (SEQ ID NO: 32) | EVQLVQSGAEVKKPGASVKVSCKASGYTFTSYWMHWVRQAPG QRLEWMGMIHPNSGSTNYNEKFKGRVTLTLDKSASTAYMELSS LRSEDTAVYYCARLKTGNSFDYWGQGTTVTVSS |
| VH13 (SEQ ID NO: 33) | EVQLVQSGAEVKKPGASVKVSCKASGYTFTSYWMHWVRQAPG QRLEWIGMIHPNSGSTNYNEKFKGKATLTLDKSASTAYMELSSL RSEDTAVYYCARLKTGNSFDYWGQGTTVTVSS |
| VH14 (SEQ ID NO: 34) | EVQLVQSGAEVKKPGASVKVSCKASGYTFTSYWMHWVKQRPG QRLEWIGMIHPNSGSTNYNEKFKGKATLTLDKSASTAYMELSSL RSEDTAVYYCARLKTGNSFDYWGQGTTVTVSS |

The corresponding heavy chain variable region in the table above was linked to the human IgG1 heavy chain constant region set forth in SEQ ID NO: 7 to form a heavy chain of a full-length antibody, and the light chain variable region was linked to the human κ light chain constant region set forth in SEQ ID NO: 8 to form a light chain of a full-length antibody.

Illustratively, the full-length sequences of the antibodies are as follows:
Chimeric antibody ch1901:
Heavy chain of ch1901: (SEQ ID NO: 35) 
EVQLMESGGGLVKPGGSLKLSCAASGFTFSDYGIHWVRQAPEMGLEWIAYISR
GSSTIYYADTVKGRFTMSRDNAKNTLFLQMTSLRSEDTAMYYCARGGYDTRN
AMDYWGQGTSVTVSSASTKGPSVFPLAPSSKSTSGGTAALGCLVKDYFPEPVTV
 
SWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK.
Light chain of ch1901: (SEQ ID NO: 36) 
DIVMTQSPSSLSVSAGEKVTMSCKSSQSLLNSGNQKNYLAWYQQKPGQPPKLLI
YGASTRASGVPDRFTGSGSGTDFTLTISSVQAEDLAIYHCQNDLYYPLTFGAGTK
LELKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNR
GEC.
Chimeric antibody ch1902:
Heavy chain of ch1902 (SEQ ID NO: 37) 
EVQLQESGAELVKPGASVKLSCKASGYIFTSYWMHWVKQRPGQGLEWIGMIHP
NSGSTNYNEKFKGKATLTLDKSSSTAYMQLSSLPSEDSAVYYCARLKTGNSFDY
WGQGTTLTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKV
EPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMHE
ALHNHYTQKSLSLSPGK.
Light chain of ch1902 (SEQ ID NO: 38)

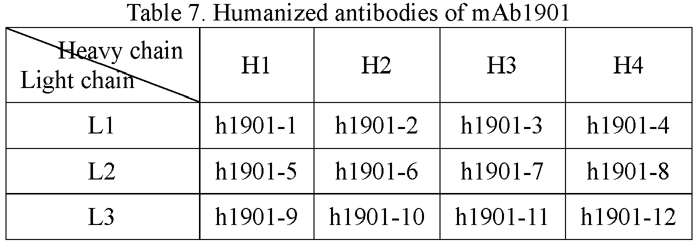

DIVLTQSPSSLTVTAGEKVTMSCKSSQSLLNSGNQKNYLTWYQQKPGQPPKLLI
YWASTRESGVPDRFTGSGSGTDFTLTISSVQAEDLAIYYCQNAYTYPFTFGSGTK
LEIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNR
GEC.

The light chain and heavy chain sequences of the full-length antibodies are as follows:

**Table 8. Light chain and heavy chain sequences of humanized antibodies of mAb1901**

| Name of light/ heavy chain (SEQ ID NO:) | Sequence |
|---|---|
| L1 (SEQ ID NO: 39) | DIVMTQSPDSLAVSLGERATINCKSSQSLLNSGNQKNYLAWYQQ KPGQPPKLLIYGASTRASGVPDRFSGSGSGTDFTLTISSLQAEDVA VYYCQNDLYYPLTFGQGTKLEIKRTVAAPSVFIFPPSDEQLKSGTA SVVCLLNNFYPREAKVQWKVDNALQSGNSQESVTEQDSKDSTY SLSSTLTLSKADYEKHKVYACEVTHQGLSSPVTKSFNRGEC |
| L2 (SEQ ID NO: 40) | DIVMTQSPDSLAVSLGERATISCKSSQSLLNSGNQKNYLAWYQQK PGQPPKLLIYGASTRASGVPDRFSGSGSGTDFTLTISSLQAEDVAV YYCQNDLYYPLTFGQGTKLEIKRTVAAPSVFIFPPSDEQLKSGTAS VVCLLNNFYPREAKVQWKVDNALQSGNSQESVTEQDSKDSTYS LSSTLTLSKADYEKHKVYACEVTHQGLSSPVTKSFNRGEC |
| L3 (SEQ ID NO: 41) | DIVMTQSPDSLAVSLGERATISCKSSQSLLNSGNQKNYLAWYQQK PGQPPKLLIYGASTRASGVPDRFSGSGSGTDFTLTISSLQAEDVAIY HCQNDLYYPLTFGQGTKLEIKRTVAAPSVFIFPPSDEQLKSGTASV VCLLNNFYPREAKVQWKVDNALQSGNSQESVTEQDSKDSTYSL SSTLTLSKADYEKHKVYACEVTHQGLSSPVTKSFNRGEC |
| H1 (SEQ ID NO: 42) | EVQLVESGGGLVQPGGSLRLSCAASGFTFSDYGIHWVRQAPGKG LEWVAYISRGSSTIYYADTVKGRFTISRDNAKNSLYLQMNSLRAE DTAVYYCARGGYDTRNAMDYWGQGTTVTVSSASTKGPSVFPLA PSSKSTSGGTAALGCLVKDYFPEPVTVSWNSGALTSGVHTFPAVL QSSGLYSLSSVVTVPSSSLGTQTYICNVNHKPSNTKVDKKVEPKS CDKTHTCPPCPAPELLGGPSVFLFPPKPKDTLMISRTPEVTCVVVD VSHEDPEVKFNWYVDGVEVHNAKTKPREEQYNSTYRVVSVLTV LHQDWLNGKEYKCKVSNKALPAPIEKTISKAKGQPREPQVYTLP PSRDELTKNQVSLTCLVKGFYPSDIAVEWESNGQPENNYKTTPPV LDSDGSFFLYSKLTVDKSRWQQGNVFSCSVMHEALHNHYTQKSL SLSPGK |
| H2 (SEQ ID NO: 43) | EVQLVESGGGLVQPGGSLRLSCAASGFTFSDYGIHWVRQAPGKG LEWVAYISRGSSTIYYADTVKGRFTISRDNAKNSLYLQMTSLRAE DTAVYYCARGGYDTRNAMDYWGQGTTVTVSSASTKGPSVFPLA PSSKSTSGGTAALGCLVKDYFPEPVTVSWNSGALTSGVHTFPAVL QSSGLYSLSSVVTVPSSSLGTQTYICNVNHKPSNTKVDKKVEPKS CDKTHTCPPCPAPELLGGPSVFLFPPKPKDTLMISRTPEVTCVVVD VSHEDPEVKFNWYVDGVEVHNAKTKPREEQYNSTYRVVSVLTV LHQDWLNGKEYKCKVSNKALPAPIEKTISKAKGQPREPQVYTLP PSRDELTKNQVSLTCLVKGFYPSDIAVEWESNGQPENNYKTTPPV LDSDGSFFLYSKLTVDKSRWQQGNVFSCSVMHEALHNHYTQKSL SLSPGK |
| H3 (SEQ ID NO: 44) | EVQLVESGGGLVQPGGSLRLSCAASGFTFSDYGIHWVRQAPGKG LEWIAYISRGSSTIYYADTVKGRFTISRDNAKNSLYLQMTSLRAED TAVYYCARGGYDTRNAMDYWGQGTTVTVSSASTKGPSVFPLAP SSKSTSGGTAALGCLVKDYFPEPVTVSWNSGALTSGVHTFPAVLQ SSGLYSLSSVVTVPSSSLGTQTYICNVNHKPSNTKVDKKVEPKSC DKTHTCPPCPAPELLGGPSVFLFPPKPKDTLMISRTPEVTCVVVDV SHEDPEVKFNWYVDGVEVHNAKTKPREEQYNSTYRVVSVLTVL HQDWLNGKEYKCKVSNKALPAPIEKTISKAKGQPREPQVYTLPP SRDELTKNQVSLTCLVKGFYPSDIAVEWESNGQPENNYKTTPPVL DSDGSFFLYSKLTVDKSRWQQGNVFSCSVMHEALHNHYTQKSLS LSPGK |
| H4 (SEQ ID NO: 45) | EVQLVESGGGLVQPGGSLRLSCAASGFTFSDYGIHWVRQAPGKG LEWVAYISRGSSTIYYADTVKGRFTMSRDNAKNSLYLQMTSLRA EDTAVYYCARGGYDTRNAMDYWGQGTTVTVSSASTKGPSVFPL APSSKSTSGGTAALGCLVKDYFPEPVTVSWNSGALTSGVHTFPAV |
| | LQSSGLYSLSSVVTVPSSSLGTQTYICNVNHKPSNTKVDKKVEPK SCDKTHTCPPCPAPELLGGPSVFLFPPKPKDTLMISRTPEVTCVVV DVSHEDPEVKFNWYVDGVEVHNAKTKPREEQYNSTYRVVSVLT VLHQDWLNGKEYKCKVSNKALPAPIEKTISKAKGQPREPQVYTL PPSRDELTKNQVSLTCLVKGFYPSDIAVEWESNGQPENNYKTTPP VLDSDGSFFLYSKLTVDKSRWQQGNVFSCSVMHEALHNHYTQK SLSLSPGK |

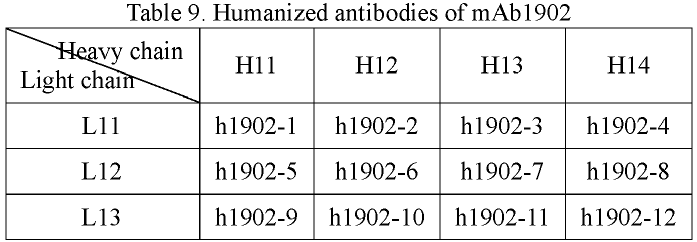

The light chain and heavy chain sequences of the full-length antibodies are as follows:

**Table 10. Light chain and heavy chain sequences of humanized antibodies of mAb 1902**

| Name of light/ heavy chain (SEQ ID NO:) | Sequence |
|---|---|
| L11 (SEQ ID NO: 46) | DIVMTQSPDSLAVSLGERATINCKSSQSLLNSGNQKNYLTWYQQKPG QPPKLLIYWASTRESGVPDRFSGSGSGTDFTLTISSLQAEDVAVYYCQN AYTYPFTFGQGTKLEIKRTVAAPSVFIFPPSDEQLKSGTASVVCLLNNF YPREAKVQWKVDNALQSGNSQESVTEQDSKDSTYSLSSTLTLSKADY EKHKVYACEVTHQGLSSPVTKSFNRGEC |
| L12 (SEQ ID NO: 47) | DIVLTQSPDSLAVSLGERATINCKSSQSLLNSGNQKNYLTWYQQKPGQ PPKLLIYWASTRESGVPDRFSGSGSGTDFTLTISSLQAEDVAVYYCQNA YTYPFTFGQGTKLEIKRTVAAPSVFIFPPSDEQLKSGTASVVCLLNNFY PREAKVQWKVDNALQSGNSQESVTEQDSKDSTYSLSSTLTLSKADYE KHKVYACEVTHQGLSSPVTKSFNRGEC |
| L13 (SEQ ID NO: 48) | DIVLTQSPDSLAVSLGERATISCKSSQSLLNSGNQKNYLTWYQQKPGQ PPKLLIYWASTRESGVPDRFSGSGSGTDFTLTISSLQAEDVAVYYCQNA YTYPFTFGQGTKLEIKRTVAAPSVFIFPPSDEQLKSGTASVVCLLNNFY PREAKVQWKVDNALQSGNSQESVTEQDSKDSTYSLSSTLTLSKADYE KHKVYACEVTHQGLSSPVTKSFNRGEC |
| H11 (SEQ ID NO: 49) | EVQLVQSGAEVKKPGASVKVSCKASGYTFTSYWMHWVRQAPGQRL EWMGMIHPNSGSTNYNEKFKGRVTITRDTSASTAYMELSSLRSEDTAV YYCARLKTGNSFDYWGQGTTVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGV EVHNAKTKPREEQYNSTYRVVSVLTVLHQDWLNGKEYKCKVSNKAL PAPIEKTISKAKGQPREPQVYTLPPSRDELTKNQVSLTCLVKGFYPSDIA VEWESNGQPENNYKTTPPVLDSDGSFFLYSKLTVDKSRWQQGNVFSC SVMHEALHNHYTQKSLSLSPGK |
| H12 (SEQ ID NO: 50) | EVQLVQSGAEVKKPGASVKVSCKASGYTFTSYWMHWVRQAPGQRL EWMGMIHPNSGSTNYNEKFKGRVTLTLDKSASTAYMELSSLRSEDTA VYYCARLKTGNSFDYWGQGTTVTVSSASTKGPSVFPLAPSSKSTSGG TAALGCLVKDYFPEPVTVSWNSGALTSGVHTFPAVLQSSGLYSLSSVV TVPSSSLGTQTYICNVNHKPSNTKVDKKVEPKSCDKTHTCPPCPAPEL LGGPSVFLFPPKPKDTLMISRTPEVTCVVVDVSHEDPEVKFNWYVDG |
| | VEVHNAKTKPREEQYNSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNV FSCSVMHEALHNHYTQKSLSLSPGK |
| H13 (SEQ ID NO: 51) | EVQLVQSGAEVKKPGASVKVSCKASGYTFTSYWMHWVRQAPGQRL EWIGMIHPNSGSTNYNEKFKGKATLTLDKSASTAYMELSSLRSEDTAV YYCARLKTGNSFDYWGQGTTVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGV EVHNAKTKPREEQYNSTYRVVSVLTVLHQDWLNGKEYKCKVSNKAL PAPIEKTISKAKGQPREPQVYTLPPSRDELTKNQVSLTCLVKGFYPSDIA VEWESNGQPENNYKTTPPVLDSDGSFFLYSKLTVDKSRWQQGNVFSC SVMHEALHNHYTQKSLSLSPGK |
| H14 (SEQ ID NO: 52) | EVQLVQSGAEVKKPGASVKVSCKASGYTFTSYWMHWVKQRPGQRL EWIGMIHPNSGSTNYNEKFKGKATLTLDKSASTAYMELSSLRSEDTAV YYCARLKTGNSFDYWGQGTTVTVSSASTKGPSVFPLAPSSKSTSGGT AALGCLVKDYFPEPVTVSWNSGALTSGVHTFPAVLQSSGLYSLSSVVT VPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGV EVHNAKTKPREEQYNSTYRVVSVLTVLHQDWLNGKEYKCKVSNKAL PAPIEKTISKAKGQPREPQVYTLPPSRDELTKNQVSLTCLVKGFYPSDIA VEWESNGQPENNYKTTPPVLDSDGSFFLYSKLTVDKSRWQQGNVFSC SVMHEALHNHYTQKSLSLSPGK |

A positive control antibody of the present disclosure is IMAB-362 (from WO2016166122):
Heavy chain of IMAB-362 (SEQ ID NO: 53) 
1 QVQLQQPGAE LVRPGASVKL SCKASGYTFT SYWINWVKQR PGQGLEWIGN
51 IYPSDSYTNY NQKFKDKATL TVDKSSSTAY MQLSSPTSED SAVYYCTRSW
101 RGNSFDYWGQ GTTLTVSSAS TKGPSVFPLA PSSKSTSGGT AALGCLVKDY
151 FPEPVTVSWN SGALTSGVHT FPAVLQSSGL YSLSSVVTVP SSSLGTQTYI
201 CNVNHKPSNT KVDKRVEPKS CDKTHTCPPC PAPELLGGPS VFLFPPKPKD
251 TLMISRTPEV TCVVVDVSHE DPEVKFNWYV DGVEVHNAKT KPREEQYNST
301 YRVVSVLTVL HQDWLNGKEY KCKVSNKALP APIEKTISKA KGQPREPQVY
351 TLPPSREEMT KNQVSLTCLV KGFYPSDIAV EWESNGQPEN NYKTTPPVLD
401 SDGSFFLYSK LTVDKSRWQQ GNVFSCSVMH EALHNHYTQK SLSLSPGK.
Light chain of IMAB-362 (SEQ ID NO: 54)

1 DIVMTQSPSSLTVTAGEKVT MSCKSSQSLL NSGNQKNYLT WYQQKPGQPP
51 KLLIYWASTR ESGVPDRFTG SGSGTDFTLTISSVQAEDLAVYYCQNDYSY
101 PFTFGSGTKLEIKRTVAAPS VFIFPPSDEQ LKSGTASVVC LLNNFYPREA
151 KVQWKVDNAL QSGNSQESVT EQDSKDSTYS LSSTLTLSKA DYEKHKVYAC
201 EVTHQGLSSP VTKSFNRGEC.

The above antibodies were cloned, expressed and purified using conventional gene cloning and recombinant expression methods.

### Test Example 1: Biological Evaluation of In Vitro Activity of Anti-Claudin18.2 Antibodies

### Test Example 1-1: Cell-Level ELISA Binding Assay

Cell-based ELISA assay was used to detect the binding properties of the Claudin18.2 antibodies. The stably transfected Claudin18.2-expressing NUGC4 cells were cultured in a 96-well cell plate (Corning, 3599). When growing at 90% density, the cells were immobilized with 4% paraformaldehyde for 1 h. The plate was washed 3 times with PBST buffer (pH 7.4 PBS containing 0.05% Tween-20), and a PBS-diluted 5% skim milk (powdered skim milk from Brightdairy) blocking solution was added at 200 µL/well. The plate was incubated in a 37 °C incubator for 2.5 h or was left to stand at 4 °C overnight (16-18 h) for blocking. After blocking, the blocking solution was removed. The plate was washed 3 times with the PBST buffer, and then a test antibody that was diluted with a sample diluent (pH 7.4 PBS containing 1% skim milk) to different concentrations was added at 50 µL/well. The plate was incubated in a 37 °C incubator for 2 h. After incubation, the plate was washed 5 times with PBST, and an HRP-labeled goat anti-human secondary antibody (Jackson Immuno Research, 109-035-003) that was diluted with the sample diluent was added at 100 µL/well. The plate was incubated at 37 °C for 1 h. The plate was washed 6 times with PBST, and then TMB chromogenic substrate (KPL, 52-00-03) was added at 50 µL/well. The plate was incubated at room temperature for 10-15 min, and the reaction was terminated by adding 1 M H₂SO₄ at 50 µL/well. The absorbance at 450 nm was read using an MD Versa Max TM microplate reader, and the binding EC₅₀ values of the Claudin18.2 antibodies to Claudin18.2 were calculated (the results are shown in the table below).

**Table 11. Binding activity of antibodies**

| Antibody | IMAB362 | ch1901 | ch1902 |
|---|---|---|---|
| Emax | 1.175 | 1.399 | 1.272 |
| EC₅₀ (nM) | 0.108 | 0.098 | 0.074 |

**Table 12-1. Binding activity of humanized antibodies of mAb 1901**

| Antibody | Emax | EC₅₀ (nM) |
|---|---|---|
| IMAB362 | 1.115 | 0.086 |
| h1901-2 | 1.039 | 0.076 |
| h1901-3 | 1.1055 | 0.22 |
| h1901-4 | 0.986 | 0.201 |
| h1901-6 | 0.937 | 0.091 |
| h1901-7 | 0.921 | 0.166 |
| h1901-8 | 1.047 | 0.091 |
| h1901-11 | 1.44 | 0.076 |
| h1901-12 | 1.22 | 0.116 |

**Table 12-2. Binding activity of humanized antibodies of mAb 1902**

| Antibody | Emax | EC₅₀ (nM) |
|---|---|---|
| IMAB362 | 0.88 | 0.187 |
| h1902-1 | 0.87 | 0.113 |
| h1902-2 | 0.88 | 0.107 |
| h1902-3 | 0.84 | 0.175 |
| h1902-4 | 0.82 | 0.087 |
| h1902-5 | 0.9 | 0.098 |
| h1902-6 | 0.78 | 0.141 |
| h1902-7 | 0.75 | 0.121 |
| h1902-8 | 0.89 | 0.132 |
| h1902-9 | 0.75 | 0.137 |
| h1902-10 | 0.89 | 0.133 |

### Test Example 1-2: Antibody Cell-Level Binding Assay

The stably transfected Claudin18.2-expressing NUGC4 cells were suspended in FACS buffer (2% fetal bovine serum (Gibco, 10099141) pH 7.4 PBS (Sigma, P4417-100TAB)) to obtain a 1×10⁶/mL cell suspension, which was then added to a 96-well round-bottom plate (Corning, 3795) at 100 µL/well. After centrifugation and removal of the supernatant, the test Claudin18.2 antibody that was diluted with FACS buffer to different concentrations was added at 50 µL/well. The plate was incubated in the dark in a 4 °C refrigerator for 1 h. The plate was washed 3 times with FACS buffer by centrifugation at 300 g, and goat anti-human IgG coated with Alexa Fluor 488 (H+L) (invitrogen, A-11013) at working concentration was added. The plate was incubated in the dark in a 4 °C refrigerator for 40 min. The plate was washed 3 times with FACS buffer by centrifugation at 300 g and tested on a BD FACS CantoII flow cytometer for geometric mean fluorescence intensity. The binding EC₅₀ values of the Claudin18.2 antibodies to the stably transfected Claudin18.2-expressing NUGC4 cells were calculated. The results are shown in FIG. 1.

### Test Example 1-3: Antibody Endocytosis Assay

A test Claudin18.2 antibody pre-labeled with DyLight 488 NHS Ester (thermofisher, 46403) was added to 1×10⁶/mL stably transfected Claudin18.2-expressing NUGC4 cells at a final concentration of 5 µg/mL. The mixture was incubated in the dark on ice for 1 h and washed 3 times with pre-cooled FACS buffer (pH 7.4 PBS, 2% fetal bovine serum) by centrifugation. After removal of the supernatant, a pre-heated complete medium was added, followed by incubation in a 37 °C cell incubator with 5% CO₂. The cells were taken out after 0, 0.5, 1, 2 and 4 h and stored in the dark on ice. After all samples were collected, they were each centrifuged at 300 g at low temperature to remove the supernatant. An elution buffer (pH 1.7 0.05 M glycine, 0.1 M sodium chloride) was added, and then the mixture was incubated at room temperature for 7 min, washed once with FACS buffer by centrifugation at 300 g, and tested on a BD FACS CantoII flow cytometer for geometric mean fluorescence intensity. The efficiency of endocytosis of the Claudin18.2 antibodies by the stably transfected Claudin18.2-expressing NUGC4 cells was calculated. The results (see FIG. 2) show that the humanized antibodies have good endocytosis efficiency.

### Test Example 1-4: Antibody Affinity Assay Based on Flow Cytometry

On the day of experiment, HEK293/hClaudin18.2 cells were collected into a U-bottomed 96-well plate at 1×10⁵ to 2×10⁵ cells per well. A human Claudin18.2 antibody that was 2× diluted serially (12 concentration points) from an initial concentration of 5 µg/mL was added, and the plate was incubated at 4 °C for 1 h. IMAB362 was used as a positive control, and a negative control with no antibody was also provided. The antibody was removed by centrifugation, and FITC anti-human IgG Fc antibody (200×) was added at 100 µL/well. The plate was incubated in the dark at 4 °C for 30 min and washed twice with PBS + 2% FBS before flow cytometry analysis. BD FACS CantoII was started and preheated, and then the BD FACSDiva software was run to start a new experiment. The HEK293/hClaudin18.2 negative control sample was tested, and the FSC and SSC voltages were adjusted to appropriate values and saved. Blank sample B and standard curve 1 were tested according to the instructions for Quantum^{™} FITC-5 MESF Kit, and the FITC voltage was adjusted to an appropriate value and saved. The samples in the U-bottomed 96-well plate were tested at the saved voltage, and data were recorded. The experimental data were analyzed using Flowjo software to obtain a Geo mean, and an MESF-Geo Mean standard curve was fit according to the instructions for Quantum^{™} FITC-5 MESF Kit. The molar concentration of the human Claudin18.2 antibody bound to HEK293/hClaudin18.2 cells and the free antibody concentration were calculated according to the concentration fluorescence value of the FITC anti-human IgG Fc antibody, and the Bmax and the dissociation constant KD of the antibody were calculated through Scatchard plots. The results are shown in Table 13.

**Table 13. Cell-level affinity of humanized antibodies**

| Antibody | IMAB362 | h1901-11 | h 1902-5 |
|---|---|---|---|
| KD (nM) | 10.2 | 6.8 | 1.64 |

### Test Example 1-5: Evaluation of ADCC Effect of Antibodies

A variety of NUGC4 cells (with high, moderate and low expression of Claudin18.2) were digested, centrifuged at 1000 rpm, resuspended, and counted. The cells were resuspended at a density of 3×10⁵ cells/mL in phenol red-free RPMI 1640 (Gibco, 11835-030) supplemented with 10% FBS (New Zealand ultra-low IgG fetal bovine serum, Gibco, 1921005PJ). Cells were added to a 96-well plate (Corning, 3903) at 25 µL/well (7500 cells/well). An antibody was diluted into the phenol red-free medium to obtain a 3× antibody dilution, which was then added to the cell plate at 25 µL/well. The plate was incubated in a 37 °C incubator with 5% CO₂ for 0.5 h.

Effector cells (FcrR3A-V158-NFAT-RE-Jurkat cells) were harvested, centrifuged at 1000 rpm, resuspended, and counted. The cells were resuspended at a density of 3×10⁶ cells/mL in phenol red-free RPMI 1640 supplemented with 10% FBS (New Zealand ultra-low IgG fetal bovine serum), and then were added to the plate at 25 µL/well (7.5×10⁴ cells/well). The plate was incubated in a 37 °C incubator with 5% CO₂ for 6 h. Bright-Glo (Promega, E2610) was added to the plate at 75 µL/well, and the chemical luminescence was detected using a microplate reader (PerkinElmer, VITOR3).

The results (see Table 14 and FIGs. 3A-3C) show that both antibodies h1901-11 and h1902-5 show high ADCC activity in the NUGC4 cells with low, moderate and high expression of Claudin18.2.

**Table 14. ADCC effect of antibodies in NUGC4 cells with varying expression levels of Claudin 18.2**

| Expression level of Claudin18.2 in NUGC4 | | h1901-11 | h 1902-5 | IMAB362 |
|---|---|---|---|---|
| Low expression | IC50 (ng/ml) | 22.42 | 35.46 | 183.4 |
| Moderate expression | IC50 (ng/ml) | 15.35 | 30.00 | 210.4 |
| High expression | IC50 (ng/ml) | 26.17 | 32.16 | 132.6 |

### Example 2: Design and Application of Signal Peptide

### Example 2-1: Design and Expression of Pertuzumab Fused with Different Signal Peptides

Antibodies fused with the following signal peptides were designed, and the combinations of signal peptide sequences are as follows:
1. MEWSWVFLFFLSVTTGVHS (SEQ ID NO: 152) and
   MSVPTQVLGLLLLWLTDARC (SEQ ID NO: 153)
2. MEWSWVFLFFLSVTTGVHS (SEQ ID NO: 152) and
   MEWSWVFLFFLSVTTGVHS (SEQ ID NO: 152)
3. MEWSWVFLFFLSLTGVHA (SEQ ID NO: 57) and
   MSVPTQVLGLLLLWLTDVRA (SEQ ID NO: 105)
4. MEWSWVFLFFLSLTGVHA (SEQ ID NO: 57) and
   MEWSWVFLFFLSLTGVHA (SEQ ID NO: 57)
5. MSVPTQVLGLLLLWLTDVRA (SEQ ID NO: 105) and
   MEWSWVFLFFLSLTGVHA (SEQ ID NO: 57)

The signal peptides in each combination described above were placed at the amino acid N terminuses of the heavy chain and the light chain of the pertuzumab antibody, respectively, and corresponding new heavy chain and light chain sequences were designed. The designed sequences are as follows:
Amino acid sequence of pertuzumab-1 antibody heavy chain containing signal peptide (SEQ ID NO: 154) 
MEWSWVFLFFLSVTTGVHSEVQLVESGGGLVQPGGSLRLSCAASGFTFTDYTM
DWVRQAPGKGLEWVADVNPNSGGSIYNQRFKGRFTLSVDRSKNTLYLQMNSL
RAEDTAVYYCARNLGPSFYFDYWGQGTLVTVSSASTKGPSVFPLAPSSKSTSGG
TAALGCLVKDYFPEPVTVSWNSGALTSGVHTFPAVLQSSGLYSLSSVVTVPSSSL
GTQTYICNVNHKPSNTKVDKKVEPKSCDKTHTCPPCPAPELLGGPSVFLFPPKPK
DTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSR
EEMTKNQVSLTCLVKGFYPSDIAVEWESNGQPENNYKTTPPVLDSDGSFFLYSK
 LTVDKSRWQQGNVFSCSVMHEALHNHYTQKSLSLSPGK
Amino acid sequence of pertuzumab-1 antibody light chain containing signal peptide (SEQ ID NO: 155) 
MSVPTQVLGLLLLWLTDARCDIQMTQSPSSLSASVGDRVTITCKASQDVSIGVA
WYQQKPGKAPKLLIYSASYRYTGVPSRFSGSGSGTDFTLTISSLQPEDFATYYCQ
QYYIYPYTFGQGTKVEIKRTVAAPSVFIFPPSDEQLKSGTASVVCLLNNFYPREA
KVQWKVDNALQSGNSQESVTEQDSKDSTYSLSSTLTLSKADYEKHKVYACEVT
HQGLSSPVTKSFNRGEC
Amino acid sequence of pertuzumab-2 antibody heavy chain containing signal peptide (SEQ ID NO: 154)
Amino acid sequence of pertuzumab-2 antibody light chain containing signal peptide (SEQ ID NO: 156) 
MEWSWVFLFFLSVTTGVHSDIQMTQSPSSLSASVGDRVTITCKASQDVSIGVAW
YQQKPGKAPKLLIYSASYRYTGVPSRFSGSGSGTDFTLTISSLQPEDFATYYCQQ
YYIYPYTFGQGTKVEIKRTVAAPSVFIFPPSDEQLKSGTASVVCLLNNFYPREAK
VQWKVDNALQSGNSQESVTEQDSKDSTYSLSSTLTLSKADYEKHKVYACEVTH
QGLSSPVTKSFNRGEC
Amino acid sequence of pertuzumab-3 antibody heavy chain containing signal peptide (SEQ ID NO: 157) 
MEWSWVFLFFLSLTGVHAEVQLVESGGGLVQPGGSLRLSCAASGFTFTDYTMD
WVRQAPGKGLEWVADVNPNSGGSIYNQRFKGRFTLSVDRSKNTLYLQMNSLR
AEDTAVYYCARNLGPSFYFDYWGQGTLVTVSSASTKGPSVFPLAPSSKSTSGGT
AALGCLVKDYFPEPVTVSWNSGALTSGVHTFPAVLQSSGLYSLSSVVTVPSSSLG
TQTYICNVNHKPSNTKVDKKVEPKSCDKTHTCPPCPAPELLGGPSVFLFPPKPKD
TLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRE
EMTKNQVSLTCLVKGFYPSDIAVEWESNGQPENNYKTTPPVLDSDGSFFLYSKL
TVDKSRWQQGNVFSCSVMHEALHNHYTQKSLSLSPGK
Amino acid sequence of pertuzumab-3 antibody light chain containing signal peptide (SEQ ID NO: 158) 
MSVPTQVLGLLLLWLTDVRADIQMTQSPSSLSASVGDRVTITCKASQDVSIGVA
WYQQKPGKAPKLLIYSASYRYTGVPSRFSGSGSGTDFTLTISSLQPEDFATYYCQ
QYYIYPYTFGQGTKVEIKRTVAAPSVFIFPPSDEQLKSGTASVVCLLNNFYPREA
KVQWKVDNALQSGNSQESVTEQDSKDSTYSLSSTLTLSKADYEKHKVYACEVT
HQGLSSPVTKSFNRGEC
Amino acid sequence of pertuzumab-4 antibody heavy chain containing signal peptide (SEQ ID NO: 157)
Amino acid sequence of pertuzumab-4 antibody light chain containing signal peptide SEQ ID NO: 159) 
MEWSWVFLFFLSLTGVHADIQMTQSPSSLSASVGDRVTITCKASQDVSIGVAWY
QQKPGKAPKLLIYSASYRYTGVPSRFSGSGSGTDFTLTISSLQPEDFATYYCQQY
 
YIYPYTFGQGTKVEIKRTVAAPSVFIFPPSDEQLKSGTASVVCLLNNFYPREAKV
QWKVDNALQSGNSQESVTEQDSKDSTYSLSSTLTLSKADYEKHKVYACEVTHQ
GLSSPVTKSFNRGEC
Amino acid sequence of pertuzumab-5 antibody heavy chain containing signal peptide (SEQ ID NO: 160) 
MSVPTQVLGLLLLWLTDVRAEVQLVESGGGLVQPGGSLRLSCAASGFTFTDYT
MDWVRQAPGKGLEWVADVNPNSGGSIYNQRFKGRFTLSVDRSKNTLYLQMNS
LRAEDTAVYYCARNLGPSFYFDYWGQGTLVTVSSASTKGPSVFPLAPSSKSTSG
GTAALGCLVKDYFPEPVTVSWNSGALTSGVHTFPAVLQSSGLYSLSSVVTVPSSS
LGTQTYICNVNHKPSNTKVDKKVEPKSCDKTHTCPPCPAPELLGGPSVFLFPPKP
KDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK
Amino acid sequence of pertuzumab-5 antibody light chain containing signal peptide (SEQ ID NO: 159)
(Note: the underlined part is signal peptide)

Based on the sequences of the pertuzumab-1 to pertuzumab-4, gene segments of each heavy chain and each light chain were synthesized. The heavy chain genes were cloned into PXC18.4 to form heavy chain plasmids, and light chain genes were cloned into PXC17.4 to form light chain plasmids. The light chain plasmid was cloned into the heavy chain plasmid through the same enzymatic digestion sites in the plasmids, and a full-length antibody plasmid was constructed. The full-length antibody plasmid was directly transfected into CHO cells by electrotransfection, and stably transfected cells containing the plasmid were obtained after screening. The stably transfected cells were cultured, and the supernatant obtained after culturing was purified by a Protein A affinity column to obtain the expression product.

### Example 2-2: Design and Expression of h1902-5 Fused with Different Signal Peptides

Antibodies fused with the following signal peptides were designed, and the combinations of signal peptide sequences are as follows:
1. MEWSWVFLFFLSVTTGVHS (SEQ ID NO: 152) and
   MEWSWVFLFFLSVTTGVHS (SEQ ID NO: 152)
2. MEWSWVFLFFLSLTGVHA (SEQ ID NO: 57) and
   MSVPTQVLGLLLLWLTDVRA (SEQ ID NO: 105)
3. MSVPTQVLGLLLLWLTDVRA (SEQ ID NO: 105) and
   MEWSWVFLFFLSLTGVHA (SEQ ID NO: 57) and

The signal peptides in each combination described above were placed at the N terminuses of the heavy chain and the light chain of the h1902-5 antibody, respectively, and corresponding new heavy chain and light chain sequences were designed. The designed sequences are as follows:
Amino acid sequence of h1902-5-1 antibody heavy chain containing signal peptide (SEQ ID NO: 161) 
MEWSWVFLFFLSVTTGVHSEVQLVQSGAEVKKPGASVKVSCKASGYTFTSYW
MHWVRQAPGQRLEWMGMIHPNSGSTNYNEKFKGRVTITRDTSASTAYMELSSL
RSEDTAVYYCARLKTGNSFDYWGQGTTVTVSSASTKGPSVFPLAPSSKSTSGGT
AALGCLVKDYFPEPVTVSWNSGALTSGVHTFPAVLQSSGLYSLSSVVTVPSSSLG
TQTYICNVNHKPSNTKVDKKVEPKSCDKTHTCPPCPAPELLGGPSVFLFPPKPKD
TLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLT
VDKSRWQQGNVFSCSVMHEALHNHYTQKSLSLSPGK
Amino acid sequence of h1902-5-1 antibody light chain containing signal peptide (SEQ ID NO: 162) 
MEWSWVFLFFLSVTTGVHSDIVLTQSPDSLAVSLGERATINCKSSQSLLNSGNQK
NYLTWYQQKPGQPPKLLIYWASTRESGVPDRFSGSGSGTDFTLTISSLQAEDVAV
YYCQNAYTYPFTFGQGTKLEIKRTVAAPSVFIFPPSDEQLKSGTASVVCLLNNFY
PREAKVQWKVDNALQSGNSQESVTEQDSKDSTYSLSSTLTLSKADYEKHKVYA
CEVTHQGLSSPVTKSFNRGEC
Amino acid sequence of h1902-5-2 antibody heavy chain containing signal peptide (SEQ ID NO: 163) 
MEWSWVFLFFLSLTGVHAEVQLVQSGAEVKKPGASVKVSCKASGYTFTSYWM
HWVRQAPGQRLEWMGMIHPNSGSTNYNEKFKGRVTITRDTSASTAYMELSSLR
SEDTAVYYCARLKTGNSFDYWGQGTTVTVSSASTKGPSVFPLAPSSKSTSGGTA
ALGCLVKDYFPEPVTVSWNSGALTSGVHTFPAVLQSSGLYSLSSVVTVPSSSLGT
QTYICNVNHKPSNTKVDKKVEPKSCDKTHTCPPCPAPELLGGPSVFLFPPKPKDT
LMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLT
VDKSRWQQGNVFSCSVMHEALHNHYTQKSLSLSPGK
Amino acid sequence of h1902-5-2 antibody light chain containing signal peptide (SEQ ID NO: 164) 
MSVPTQVLGLLLLWLTDVRADIVLTQSPDSLAVSLGERATINCKSSQSLLNSGNQ
KNYLTWYQQKPGQPPKLLIYWASTRESGVPDRFSGSGSGTDFTLTISSLQAEDVA
VYYCQNAYTYPFTFGQGTKLEIKRTVAAPSVFIFPPSDEQLKSGTASVVCLLNNF
YPREAKVQWKVDNALQSGNSQESVTEQDSKDSTYSLSSTLTLSKADYEKHKVY
ACEVTHQGLSSPVTKSFNRGEC
Amino acid sequence of h1902-5-3 antibody heavy chain containing signal peptide (SEQ ID NO: 165) 
MSVPTQVLGLLLLWLTDVRAEVQLVQSGAEVKKPGASVKVSCKASGYTFTSY
WMHWVRQAPGQRLEWMGMIHPNSGSTNYNEKFKGRVTITRDTSASTAYMELS
 
SLRSEDTAVYYCARLKTGNSFDYWGQGTTVTVSSASTKGPSVFPLAPSSKSTSG
GTAALGCLVKDYFPEPVTVSWNSGALTSGVHTFPAVLQSSGLYSLSSVVTVPSSS
LGTQTYICNVNHKPSNTKVDKKVEPKSCDKTHTCPPCPAPELLGGPSVFLFPPKP
KDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SRDELTKNQVSLTCLVKGFYPSDIAVEWESNGQPENNYKTTPPVLDSDGSFFLYS
KLTVDKSRWQQGNVFSCSVMHEALHNHYTQKSLSLSPGK
Amino acid sequence of h1902-5-3 antibody light chain containing signal peptide (SEQ ID NO: 166) 
MEWSWVFLFFLSLTGVHADIVLTQSPDSLAVSLGERATINCKSSQSLLNSGNQKN
YLTWYQQKPGQPPKLLIYWASTRESGVPDRFSGSGSGTDFTLTISSLQAEDVAVY
YCQNAYTYPFTFGQGTKLEIKRTVAAPSVFIFPPSDEQLKSGTASVVCLLNNFYP
REAKVQWKVDNALQSGNSQESVTEQDSKDSTYSLSSTLTLSKADYEKHKVYAC
EVTHQGLSSPVTKSFNRGEC
(Note: the underlined part is signal peptide)

Based on the sequences of the h1902-5-1 or h1902-5-2 described above, gene segments of each heavy chain and each light chain were synthesized and constructed on an expression vector to obtain a full-length antibody plasmid. The full-length antibody plasmid was directly transfected into CHO cells by electrotransfection, and stably transfected cells containing the plasmid were obtained after screening. The stably transfected cells were cultured, and the supernatant obtained after culturing was purified by a Protein A affinity column to obtain the expression product.

### Example 2-3: Design and Expression of FcRn Fused with Different Signal Peptides

Amino acid sequences of the heavy chain and the light chain of the anti-FcRn antibody are as follows:
Heavy chain (SEQ ID NO: 167) 
EVQLVQSGAEVKKPGESLKISCKGSGYNFNKHYIAWVRQMPGKGLEWMGIIYP
DNSNTIYSPSFQGQVTISADKSISTAYLQWSSLKASDTAMYYCARFGGPTFAQW
YFDYWGQGTLVTVSSASTKGPSVFPLAPCSRSTSESTAALGCLVKDYFPEPVTVS
WNSGALTSGVHTFPAVLQSSGLYSLSSVVTVPSSSLGTKTYTCNVDHKPSNTKV
DKRVESKYGPPCPPCPAPEFLGGPSVFLFPPKPKDTLMISRTPEVTCVVVDVSQE
DPEVQFNWYVDGVEVHNAKTKPREEQFNSTYRVVSVLTVLHQDWLNGKEYK
CKVSNKGLPSSIEKTISKAKGQPREPQVYTLPPSQEEMTKNQVSLTCLVKGFYPS
DIAVEWESNGQPENNYKTTPPVLDSDGSFFLYSRLTVDKSRWQEGNVFSCSVMH
EALHNHYTQKSLSLSLGK
Light chain (SEQ ID NO: 168)

NFMLTQPHSVSESPGKTVTISCTGSSGSIASNYVQWYQQRPGSSPTTVIYEDNQR
ASGVPDRFSGSIDSSSNSASLTISGLKTEDEADYYCQSYDSSSHNWVFGGGTKLT
VLGQPKANPTVTLFPPSSEELQANKATLVCLISDFYPGAVTVAWKADGSPVKAG
VETTKPSKQSNNKYAASSYLSLTPEQWKSHRSYSCQVTHEGSTVEKTVAPTECS

Antibodies fused with the following signal peptides were designed, and the combinations of signal peptide sequences are as follows:
1. MEWSWVFLFFLSVTTGVHS (SEQ ID NO: 152) and
   MEWSWVFLFFLSVTTGVHS (SEQ ID NO: 152)
2. MEWSWVFLFFLSLTGVHA (SEQ ID NO: 57) and
   MEWSWVFLFFLSLTGVHA (SEQ ID NO: 57)

The designed sequences are as follows:
Amino acid sequence of FcRn-1 antibody heavy chain containing signal peptide (SEQ ID NO: 169) 
MEWSWVFLFFLSVTTGVHSEVQLVQSGAEVKKPGESLKISCKGSGYNFNKHYI
AWVRQMPGKGLEWMGIIYPDNSNTIYSPSFQGQVTISADKSISTAYLQWSSLKA
SDTAMYYCARFGGPTFAQWYFDYWGQGTLVTVSSASTKGPSVFPLAPCSRSTS
ESTAALGCLVKDYFPEPVTVSWNSGALTSGVHTFPAVLQSSGLYSLSSVVTVPSS
SLGTKTYTCNVDHKPSNTKVDKRVESKYGPPCPPCPAPEFLGGPSVFLFPPKPKD
TLMISRTPEVTCVVVDVSQEDPEVQFNWYVDGVEVHNAKTKPREEQFNSTYRV
VSVLTVLHQDWLNGKEYKCKVSNKGLPSSIEKTISKAKGQPREPQVYTLPPSQE
EMTKNQVSLTCLVKGFYPSDIAVEWESNGQPENNYKTTPPVLDSDGSFFLYSRLT
VDKSRWQEGNVFSCSVMHEALHNHYTQKSLSLSLGK
Amino acid sequence of FcRn-1 antibody light chain containing signal peptide (SEQ ID NO: 170) 
MEWSWVFLFFLSVTTGVHSNFMLTQPHSVSESPGKTVTISCTGSSGSIASNYVQ
WYQQRPGSSPTTVIYEDNQRASGVPDRFSGSIDSSSNSASLTISGLKTEDEADYY
CQSYDSSSHNWVFGGGTKLTVLGQPKANPTVTLFPPSSEELQANKATLVCLISDF
YPGAVTVAWKADGSPVKAGVETTKPSKQSNNKYAASSYLSLTPEQWKSHRSYS
CQVTHEGSTVEKTVAPTECS
Amino acid sequence of FcRn-2 antibody heavy chain containing signal peptide (SEQ ID NO: 171) 
MEWSWVFLFFLSLTGVHAEVQLVQSGAEVKKPGESLKISCKGSGYNFNKHYIA
WVRQMPGKGLEWMGIIYPDNSNTIYSPSFQGQVTISADKSISTAYLQWSSLKAS
DTAMYYCARFGGPTFAQWYFDYWGQGTLVTVSSASTKGPSVFPLAPCSRSTSE
STAALGCLVKDYFPEPVTVSWNSGALTSGVHTFPAVLQSSGLYSLSSVVTVPSSS
LGTKTYTCNVDHKPSNTKVDKRVESKYGPPCPPCPAPEFLGGPSVFLFPPKPKDT
LMISRTPEVTCVVVDVSQEDPEVQFNWYVDGVEVHNAKTKPREEQFNSTYRVV
SVLTVLHQDWLNGKEYKCKVSNKGLPSSIEKTISKAKGQPREPQVYTLPPSQEE
MTKNQVSLTCLVKGFYPSDIAVEWESNGQPENNYKTTPPVLDSDGSFFLYSRLT
VDKSRWQEGNVFSCSVMHEALHNHYTQKSLSLSLGK
Amino acid sequence of FcRn-2 antibody light chain containing signal peptide (SEQ ID NO: 172)

MEWSWVFLFFLSLTGVHANFMLTQPHSVSESPGKTVTISCTGSSGSIASNYVQW
YQQRPGSSPTTVIYEDNQRASGVPDRFSGSIDSSSNSASLTISGLKTEDEADYYC
QSYDSSSHNWVFGGGTKLTVLGQPKANPTVTLFPPSSEELQANKATLVCLISDFY

PGAVTVAWKADGSPVKAGVETTKPSKQSNNKYAASSYLSLTPEQWKSHRSYSC
QVTHEGSTVEKTVAPTECS

Based on the sequences of the FcRn-1 and FcRn-2 described above, gene segments of each heavy chain and each light chain were synthesized and constructed on an expression vector to obtain a full-length antibody plasmid. The full-length antibody plasmid was directly transfected into CHO cells by electrotransfection, and stably transfected cell pool containing the plasmid was obtained after screening. The stably transfected cell pool was cultured, and the supernatant obtained after culturing was purified by a Protein A affinity column to obtain the expression product.

### Test Example 2: Terminal Heterogeneity of Expression Products with Different Signal Peptides

### Test Example 2-1: Detection of Molecular Weight after Deglycosylation and Reduction and Amino Acid Sequence of Pertuzumab Fused with Different Signal Peptides

N-glycosyl carried by pertuzumab-1, pertuzumab-2, pertuzumab-3 and pertuzumab-4 samples was cut off by PNGase F glycosidase (NEB, P0708), and then the samples were each reduced into light and heavy chains by DTT (Sigma, 43815). Finally, the molecular weight after deglycosylation and reduction of each sample was detected by LC-MS (Waters, ACQUITY UPLC H-Class/XeVo G2-XS QTOF), and data processing and analysis were performed using UNIFI software.

The detection results of pertuzumab-1 and pertuzumab-3 are shown in FIGs. 4A-4D: 4.7% signal peptide amino acid (RC) residue was detected in the light chain of the pertuzumab-1 sample, while the light chain of the pertuzumab-3 sample had no signal peptide amino acid residue. No signal peptide amino acid residue was detected in the heavy chains of both pertuzumab-1 and pertuzumab-3, and only a small amount of glycated modification was present.

Pertuzumab-1 was subjected to urea denaturation, DTT was then added for reduction into light and heavy chains, and then Glu C enzyme (Promega, V1651) was added for enzymatic digestion. The molecular weight data of the resulting sample was collected by LC-MS and then analyzed using UNIFI software to obtain the amino acid sequence information of the sample.

The results showed that for pertuzumab-1, abnormal peaks that appeared in the detection of the molecular weight after deglycosylation and reduction were confirmed to be RC amino acid residue through peptide mapping analysis and verification. Specific results are shown in Table 15.

**Table 15. Peptide mapping assay data of pertuzumab-1 light chain N-terminal signal peptide**

| Sample | Terminal extension residue | Intensity | Theoretical molecular weight (Da) | Measured molecular weight (Da) | Retention time (min) |
|---|---|---|---|---|---|
| | | | | | |
| Pertuzumab-1 | None | 205984816 | 1878.8862 | 1878.8917 | 30.41 |
| | C | 20145608 | 2038.9168 | 2038.9145 | 30.17 |

| | | | | | |
|---|---|---|---|---|---|
| Note: In this test example, Glu C enzyme is used for enzymatic digestion to cut off the amino acid R in the sequence RC, and the residual amino acid C indicates RC amino acid residue. | | | | | |

The detection results of pertuzumab-2 and pertuzumab-4 are shown in FIGs. 5A-5D: 5.8% signal peptide amino acid (VHS) residue was detected in the light chain of the pertuzumab-2 sample, while the light chain of the pertuzumab-4 sample had no signal peptide amino acid residue. No signal peptide amino acid residue was detected in the heavy chains of both antibodies, and only a small amount of glycated modification was present.

Pertuzumab-2 was subjected to urea denaturation, DTT was then added for reduction into light and heavy chains, and then Glu C enzyme was added for enzymatic digestion. The molecular weight data of the resulting sample was collected by LC-MS and then analyzed using UNIFI software to obtain the amino acid sequence information of the sample.

The results showed that for pertuzumab-2, abnormal peaks that appeared in the detection of the molecular weight after deglycosylation and reduction were confirmed to be VHS amino acid residue through peptide mapping analysis and verification. Specific results are shown in Table 16.

**Table 16. Peptide mapping assay data of pertuzumab-1 light chain N-terminal signal peptide**

| Sample | Terminal extension residue | Intensity | Theoretical molecular weight (Da) | Measured molecular weight (Da) | Retention time (min) |
|---|---|---|---|---|---|
| Pertuzumab-2 | None | 177359008 | 1878.8883 | 1878.8862 | 30.4 |
| | VHS | 15853681 | 2202.0450 | 2202.0455 | 28.4 |

### Test Example 2-2: Detection of Molecular Weight after Deglycosylation and Reduction and Amino Acid Sequence of h1902-5 Fused with Different Signal Peptides

N-glycosyl carried by h1902-5-1 and h1902-5-2 samples was cut off by PNGase F glycosidase (NEB, P0708), and then the samples were each reduced into light and heavy chains by DTT (Sigma, 43815). Finally, the molecular weight after deglycosylation and reduction of each sample was detected by LC-MS (Waters, ACQUITY UPLC H-Class/XeVo G2-XS QTOF), and data processing and analysis were performed using UNIFI software.

The results are shown in FIGs. 6A-6D. 3.1% signal peptide amino acid (VHS) residue was detected in the heavy chain of the h1902-5-1 sample, while the heavy chain of the h1902-5-2 sample had no signal peptide amino acid residue. No signal peptide amino acid residue was detected in the light chains of both antibodies, and only a small amount of glycated modification was present.

h1902-5-1 was subjected to urea denaturation, DTT was then added for reduction into light and heavy chains, and then Glu C enzyme was added for enzymatic digestion. The molecular weight data of the resulting sample was collected by LC-MS and then analyzed using UNIFI software to obtain the amino acid sequence information of the sample.

The results showed that for h1902-5-1, abnormal peaks that appeared in the detection of the molecular weight after deglycosylation and reduction were confirmed to be VHS amino acid residue through peptide mapping analysis and verification. Specific results are shown in Table 17 below.

**Table 17. Peptide mapping assay data of h1902-5-1 heavy chain N-terminal signal peptide**

| Sample | Terminal extension residue | Intensity | Theoretical molecular weight (Da) | Measured molecular weight (Da) | Retention time (min) |
|---|---|---|---|---|---|
| h1902-5-1 | None | 150012880 | 1286.6952 | 1286.6951 | 25.53 |
| | VHS | 6470631 | 1609.8517 | 1609.8544 | 25.11 |

### Test Example 2-3: Detection of Molecular Weight after Deglycosylation and Reduction and Amino Acid Sequence of Anti-FcRn Antibody Fused with Different Signal Peptides

N-glycosyl carried by FcRn-1 and FcRn-2 samples was cut off by PNGase F glycosidase (NEB, P0708), and then the samples were each reduced into light and heavy chains by DTT (Sigma, 43815). Finally, the molecular weight after deglycosylation and reduction of each sample was detected by LC-MS (Waters, ACQUITY UPLC H-Class/XeVo G2-XS QTOF), and data processing and analysis were performed using UNIFI software.

The results are shown in FIGs. 7A-7D. 3.4% signal peptide amino acid (VHS) residue was detected in the heavy chain of the FcRn-1 sample, while the heavy chain of the FcRn-2 sample had no signal peptide amino acid residue. No signal peptide amino acid residue was detected in the light chains of both antibodies, and only a small amount of glycated modification was present.

FcRn-1 was subjected to urea denaturation, DTT was then added for reduction into light and heavy chains, and then Glu C enzyme was added for enzymatic digestion. The molecular weight data of the resulting sample was collected by LC-MS and then analyzed using UNIFI software to obtain the amino acid sequence information of the sample.

The results showed that for FcRn-1, abnormal peaks that appeared in the detection of the molecular weight after deglycosylation and reduction were confirmed to be VHS amino acid residue through peptide mapping analysis and verification. Specific results are shown in the table below.

**Table 18. Peptide mapping assay data of FcRn heavy chain N-terminal signal peptide**

| Sample | Terminal extension residue | Intensity | Theoretical molecular weight (Da) | Measured molecular weight (Da) | Retention time (min) |
|---|---|---|---|---|---|
| FcRn-1 | None | 409437184 | 1286.695 | 1286.69506 | 25.69 |
| | VHS | 36230248 | 1609.8521 | 1609.85441 | 25.3 |

Although the foregoing invention has been described in detail by way of drawings and examples for clarity of understanding, the description and examples should not be construed as limiting the scope of the present disclosure. The disclosures of all patents and scientific literature cited herein are clearly incorporated by reference in their entirety.

## Claims

1. A method for reducing N-terminal heterogeneity of a heavy chain of an antibody and/or a light chain of an antibody, comprising
culturing a host cell, wherein the host cell comprises
(1) a first polynucleotide encoding the heavy chain of the antibody and a first signal peptide operably linked to the N-terminus of the heavy chain; wherein the first signal peptide comprises an amino acid sequence of SEQ ID NO: 55 or SEQ ID NO: 56; and/or
(2) a second polynucleotide encoding the light chain of the antibody and a second signal peptide operably linked to the N-terminus of the light chain; wherein the second signal peptide comprises an amino acid sequence of SEQ ID NO: 55 or SEQ ID NO: 56; and expressing the heavy chain of the antibody and/or the light chain of the antibody.

2. The method according to claim 1, wherein the heavy chain of the antibody and/or the light chain of the antibody obtained through expression has a terminal extension ratio of less than 3%; preferably, the heavy chain of the antibody and/or the light chain of the antibody obtained through expression has a terminal extension ratio of less than 1%.

3. The method according to claim 1 or 2, wherein the first signal peptide or the second signal peptide each independently comprises an amino acid sequence of SEQ ID NO: 57, SEQ ID NO: 58, SEQ ID NO: 59, SEQ ID NO: 93, SEQ ID NO: 94, SEQ ID NO: 95, SEQ ID NO: 99, SEQ ID NO: 100, SEQ ID NO: 101, SEQ ID NO: 105, SEQ ID NO: 106, SEQ ID NO: 107, SEQ ID NO: 140, SEQ ID NO: 141, SEQ ID NO: 142, SEQ ID NO: 146, SEQ ID NO: 147 or SEQ ID NO: 148;
preferably, the first signal peptide comprises the amino acid sequence of SEQ ID NO: 57 or SEQ ID NO: 105, and/or the second signal peptide comprises the amino acid sequence of SEQ ID NO: 57 or SEQ ID NO: 105.

4. The method according to any one of claims 1 to 3, wherein the antibody is a murine antibody, a chimeric antibody, a humanized antibody, a human antibody, an affinity-matured antibody or a multispecific antibody.

5. The method according to any one of claims 1 to 4, wherein the antibody is selected from the group consisting of an anti-TLR7 antibody, an anti-HER2 (ErbB2) antibody, an anti-Claudin18.2 antibody, an anti-EGFR antibody, an anti-B7H3 antibody, an anti-c-Met antibody, an anti-HER3 (ErbB3) antibody, an anti-HER4 (ErbB4) antibody, an anti-CD3 antibody, an anti-CD20 antibody, an anti-CD22 antibody, an anti-CD30 antibody, an anti-CD33 antibody, an anti-CD38 antibody, an anti-CD44 antibody, an anti-CD47 antibody, an anti-CD56 antibody, an anti-CD70 antibody, an anti-CD73 antibody, an anti-CD105 antibody, an anti-CEA antibody, an anti-A33 antibody, an anti-Cripto antibody, an anti-SOST antibody, an anti-EphA2 antibody, an anti-G250 antibody, an anti-MUCl antibody, an anti-Lewis Y antibody, an anti-VEGFR antibody, an anti-GPNMB antibody, an anti-thrombin antibody, an anti-Aβ antibody, an anti-Integrin antibody, an anti-ANGPTL3 antibody, an anti-PSMA antibody, an anti-PD-1 antibody, an anti-PD-L1 antibody, an anti-LAG3 antibody, an anti-IL-5 antibody, an anti-IL-15 antibody, an anti-IL-4R antibody, an anti-IL-6R antibody, an anti-TIGHT antibody, an anti-Tenascin-C antibody, an anti-SLC44A4 antibody, an anti-PCSK9 antibody, an anti-EpCAM antibody, an anti-CTGF antibody, an anti-TSLP antibody, an anti-CEA antibody, an anti-Mesothelin antibody and an anti-FcRn antibody;
preferably, the antibody is selected from the group consisting of trastuzumab, pertuzumab, nimotuzumab, enoblituzumab, emibetuzumab, inotuzumab, pinatuzumab, brentuximab, gemtuzumab, bivatuzumab, lorvotuzumab, cBR96, glematumamab, an anti-Claudin18.2 antibody and an anti-FcRn antibody, wherein a heavy chain and a light chain of the anti-Claudin18.2 antibody comprise amino acid sequences of SEQ ID NO: 49 and SEQ ID NO: 47, respectively; a heavy chain and a light chain of the anti-FcRn antibody comprise amino acid sequences of SEQ ID NO: 167 and SEQ ID NO: 168, respectively.

6. A polypeptide, comprising a signal peptide comprising an amino acid sequence of SEQ ID NO: 55 or SEQ ID NO: 56.

7. The polypeptide according to claim 6, wherein the polypeptide further comprises a heterologous polypeptide operably linked to the signal peptide;
preferably, the heterologous polypeptide is a heavy chain of an antibody or a light chain of an antibody;
more preferably, the signal peptide is operably linked to the N-terminus of the heavy chain of the antibody or the light chain of the antibody.

8. The polypeptide according to claim 6 or 7, wherein the signal peptide comprises an amino acid sequence of SEQ ID NO: 57, SEQ ID NO: 58, SEQ ID NO: 59, SEQ ID NO: 93, SEQ ID NO: 94, SEQ ID NO: 95, SEQ ID NO: 99, SEQ ID NO: 100, SEQ ID NO: 101, SEQ ID NO: 105, SEQ ID NO: 106, SEQ ID NO: 107, SEQ ID NO: 140, SEQ ID NO: 141, SEQ ID NO: 142, SEQ ID NO: 146, SEQ ID NO: 147 or SEQ ID NO: 148;
preferably, the signal peptide comprises the amino acid sequence of SEQ ID NO: 57 or SEQ ID NO: 105.

9. The polypeptide according to any one of claims 6 to 8, wherein the polypeptide comprises an amino acid sequence selected from the group consisting of SEQ ID NO: 157, SEQ ID NO: 158, SEQ ID NO: 159, SEQ ID NO: 160, SEQ ID NO: 163, SEQ ID NO: 164, SEQ ID NO: 165, SEQ ID NO: 166, SEQ ID NO: 171 and SEQ ID NO: 172.

10. A nucleic acid molecule, encoding the polypeptide according to any one of claims 6 to 9.

11. A host cell, comprising the nucleic acid molecule according to claim 10.

12. The host cell according to claim 11, wherein the host cell is a eukaryotic host cell; preferably, the eukaryotic host cell is a CHO cell or a yeast.

13. A composition, comprising an anti-Claudin18.2 antibody, wherein a heavy chain and a light chain of the anti-Claudin18.2 antibody comprise amino acid sequences of SEQ ID NO: 49 and SEQ ID NO: 47, respectively; the anti-Claudin18.2 antibody in the composition has terminal heterogeneity, and the heavy chain and/or the light chain of the anti-Claudin18.2 antibody has a terminal extension ratio of less than 3%;
preferably, the heavy chain of the anti-Claudin18.2 antibody has a terminal extension ratio of less than 1%.

14. A composition, comprising an anti-FcRn antibody, wherein a heavy chain and a light chain of the anti-FcRn antibody comprise amino acid sequences of SEQ ID NO: 167 and SEQ ID NO: 168, respectively; the anti-FcRn antibody in the composition has terminal heterogeneity, and the heavy chain and/or the light chain of the anti-FcRn antibody has a terminal extension ratio of less than 3%;
preferably, the heavy chain of the anti-FcRn antibody has a terminal extension ratio of less than 1%.

15. The composition according to claim 13 or 14, wherein the composition was prepared by the method according to any one of claims 1 to 3.
